# EUROPEAN PATENT SPECIFICATION

(11) **EP 2 004 857 B1**
(45) Date of publication and mention of the grant of the patent: **06.06.2012**
(21) Application number: 07732350.9
(22) Date of filing: 10.04.2007
(51) Int. Cl.: C12Q 1/68

(54) **BREAST CANCER MARKERS**
BRUSTKREBSMARKER
MARQUEURS DE CANCER DU SEIN

(30) Priority: 06.04.2006 GB 0606954
(43) Date of publication of application: 24.12.2008
(73) Proprietor: Randox Laboratories Ltd., Crumlin, County Antrim BT29 4QY (GB)
(72) Inventor: CROCKARD, Martin Andrew, Co. Antrim BT29 4QY (GB); LAMONT, John Victor, Co. Antrim BT29 4QY (GB); FITZGERALD, Stephen Peter, Co. Antrim BT29 4QY (GB)
(74) Representative: Jappy, John William Graham
(86) International application number: PCT/GB2007/001306
(87) International publication number: WO 2007/128984

(56) References cited:
- EP-A- 1 394 267
- EP-A- 1 580 265
- WO-A-01/38490
- WO-A-01/94629
- WO-A-2004/016225
- WO-A-2004/048938
- WO-A-2005/005601
- WO-A-2005/024060
- TAKEUCHI T ET AL: "H-cadherin expression in breast cancer." HISTOPATHOLOGY JUL 1999, vol. 35, no. 1, July 1999 (1999-07), pages 87-88, XP002483919 ISSN: 0309-0167

### SEQUENCE LISTING

<110> Randox Laboratories Ltd.
<120> Breast Cancer Markers
<130> JWJ01255WO
<150> GB0606954.6
   <151> 2006-04-06
<160> 33
<170> PatentIn version 3.3
<210> 1
   <211> 120
   <212> DNA
   <213> Homo sapiens
<400> 1
   aattattctc atgcagtgct ctacttgact tttatctttg aattcacaac taaaaaccca     60
   tagcccagaa atctaaaaaa agtaatttta gtggagcctt tgaaaataaa agaccattgg    120
<210> 2
   <211> 261
   <212> DNA
   <213> Homo sapiens
<400> 2
   cttctcaacg ccattacact cttctggccc ctaaaaatac tcattttaat taataattgc     60
   cagaggaaag aagaaatatt tgagtaagac atgtagtttc cctttagagt cattttttaa    120
   tccagcaaat tgcaaagcca ggccaggcac ggtggctgac acctgtaatc ccagcacttt    180
   gagaagttag ataatttgag ctcaggagtt tgagaccagc ctgggcaaca tggtgaaacc    240
   ccatctcttc aaaaaaaaaa a                                               261
<210> 3
   <211> 115
   <212> DNA
   <213> Homo sapiens
<400> 3
   ttttgacgtt ctctgccaag gatttagagc tttcgttgaa ctaacataaa aggagtgcga 60
   gtcttagtag agatgttccg tgtgtgccgc ccgtgctctg aactgcgttt ccacc 115
<210> 4
   <211> 275
   <212> DNA
   <213> Homo sapiens
<400> 4
   ctggttccat tccatcaaag gccctcttga gagtctatcc agggacccat tgttttactt 60
   taacagacca gaaaagatgt ttgttttcca tgtcattacc cccaggggat accgaatgtg 120
   tgggtagaaa tttctctgta gattaaaaat cagattttta catggattca acaaaggagc 180
   gtcacttgga tttttgtttt catccatgaa tgtagctgct tctgtgtaaa atgccatttt 240
   gctattaaaa atcaattcac gctggaaaaa aaaaa 275
<210> 5
   <211> 321
   <212> DNA
   <213> Homo sapiens
<400> 5
   agcttacggg gtgacaggtt tagagggtgg ccaggccatg caggagggtg acagtttggt 60
   tacccagcaa ggcattttga ccctgaagat attttgtact aaaagttaat tttcctttat 120
   tgaataatgg tcttcagaaa aagtaaaact tagagcagaa tggccaaagt tataattggt 180
   ctttcagatt ttttcatatg gacaagaaac tgacccacga attataaaat ccatgtggaa 240
   aagaattgat ccaaatcaat gtaacttcaa gaaaatgtag aaaactttat aaaggagtaa 300
   attggcttta ttctcttgat g 321
<210> 6
   <211> 324
   <212> DNA
   <213> Homo sapiens
<400> 6
   gcttttgagg tgtagacaag tttcattgct ctaagacctg taatccaggc tccctgcctc 60
   ttatttaaac agttaccttt gggaatagca gtgtacacag agtcacttta aactcatttc 120
   ctgtctccct catggtgcct gccacgcata gagctagcta ctgaatcgga acttagtaaa 180
   cacgtgtgga ttgatttagc taactttaca tggttccttg ctgtggcaaa gtgactgttt 240
   tgtttctagt ccatgatatg tgtgctaatt tctatcaacc atgtccgcta cctttttaaa 300
   aaagacagat ggtaaaaaaa aaaa 324
<210> 7
   <211> 176
   <212> DNA
   <213> Homo sapiens
<400> 7
   gcttaactga ggggacaagt gacaatttag cagagaggca agatttgaat ccagactgtc 60
   ttccagactc aggacctacc ttaaaataat atctgagttg cctatggagg cagacctgcc 120
   tgcaaagccc agcactcagc aagtgctcaa taaatatttg atttgaaaaa aaaaaa 176
<210> 8
   <211> 966
   <212> DNA
   <213> Homo sapiens
<400> 8
   ggtgacctgg tcacagcaca ggcttctttt gtgttctgat ggtgatattt gaccaacaat 60
   aatcacaacc actctgcaca tttactatat gcaaatttct gttcatctct gatctccttt 120
   cctgcttcaa acacccagtg agcttggagt tttaccaaag gggaaaccag gacttaggaa 180
   gtaatgaggg atgttgttga agacttcttg tcattagacc aggttattgg tccaaagtaa 240
   tgaaggtaaa tctagtccct gaggacaggc tcactaccag gctataatgg gaatttgtgc 300
   taattagaga aactttccct ttctgctgcg ctttgtccca aggacccaaa tttggccagt 360
   gttccccctc tctataacca aaatatgtct tatttccctc tcttttaata ccaggagggg 420
   tccctctact ccagtcgaaa gacaatcttt gagtgctttg acccgaatac atcctcccac 480
   atctcagggt cccacaactt ccccatcagg gcccaacccc agcatagaag ccattaaagc 540
   tgacattcaa catcctttgt gattctgcta ctcttctaat gatggctggg gcccaatctt 600
   ctattctttc tgccctgagg ctccaggaaa taagaataag gactcaggtt ccttccaaaa 660
   caccctatga tgtttgcgtc atctcctggg tcagtgctga gtcacggatt tgttcatgtc 720
   cctgcctgcc agaagaggaa agacacgaaa tagaagacaa acaatgtccc caatcatcag 780
   agatgtgtgg aaggtgcata gtcacgtcca ctgaaaactt gctcacgcat gcacctgcag 840
   ttgcacattg agggatcatg tgatcaagga catttttcac aaaaccagtg tcattgtcca 900
   cttaaatttt aagtgttcaa atcactaaat ttcataaagt tattaaaata atttcctcat 960
   atcatc 966
<210> 9
   <211> 543
   <212> DNA
   <213> Homo sapiens
<400> 9
   atcttgggag actcagtaca agtagatcaa cccatctcct ctctacattt tcagggaaat 60
   ttggctcaga gtccaaggct gaaccttgtg atgaggaaac cttgctggaa gctgaccttt 120
   actgtagggg ccctgattca atatcctata tccacaaaaa atgcacatac agggcagcga 180
   ggttctagcc ttcccctctg ccatcttgat ctgtgagggt ctccttggaa aagtctatgt 240
   gtctcctaca gtgccaagca ggggaggaat cagaattagt ccaggtggca gcatcctttt 300
   atgatatgat ataaactcaa caaattaggt atagaaggaa tgtaccacaa cacaataaag 360
   gccatatatg acaagcccac aggtgatatc acacttaaca ttgaaaagtt gaaagccttt 420
   gcccgaagat taggaacaag acaatgatgc ccacccttgc catttctatt caacatagta 480
   ctggaagtcc cagcaagagc aattaggcaa ggaaaataaa taaaaaacat ctacattgga 540
   aag 543
<210> 10
   <211> 516
   <212> DNA
   <213> Homo sapiens
<400> 10
   acaattctag cgctgcctct cccggctctt cactgcacgc agcctgaggg cgtcgttgtc 60
   tgtgctgcac cctggtgctc agtgggtctc cagctggcac agtcaccacc actcccagat 120
   gggctctgcc agggacaggg atggagcaga ctgcatgggc ctggcgtgca ggtgaccagg 180
   tagaggcagc tcagctgtgt ctgaacagct tttcccatcg ctttcccggt cgacattcat 240
   ggcagccgct gcgtccaatg acaggcacag tcaattcttt gagacagaaa gggaaggaca 300
   acactaatgt ccccatgaac cccagaaaca tgaaggaggg gcacacgccc acagtagaac 360
   atgtggaagc tgagtgtggc agtgtaaagg gcgatggagc ccccgggccc agcatgtttc 420
   gcgctgcctg cttgtgttct gatgggcggt aactgaaggt ccaccaggaa gtgcctggca 480
   ccccccctct cgctcgtgaa agatgcagat cagagc 516
<210> 11
   <211> 734
   <212> PRT
   <213> Homo sapiens
<400> 11
   Met Leu Leu Trp Leu Leu Leu Leu Ile Leu Thr Pro Gly Arg Glu Gln
   1               5                   10                  15
   Ser Gly Val Ala Pro Lys Ala Val Leu Leu Leu Asn Pro Pro Trp Ser
               20                  25                  30
   Thr Ala Phe Lys Gly Glu Lys Val Ala Leu Ile Cys Ser Ser Ile Ser
           35                  40                  45
   His Ser Leu Ala Gln Gly Asp Thr Tyr Trp Tyr His Asp Glu Lys Leu
       50                  55                  60
   Leu Lys Ile Lys His Asp Lys Ile Gln Ile Thr Glu Pro Gly Asn Tyr
   65                  70                  75                  80
   Gln Cys Lys Thr Arg Gly Ser Ser Leu Ser Asp Ala Val His Val Glu
                   85                  90                  95
   Phe Ser Pro Asp Trp Leu Ile Leu Gln Ala Leu His Pro Val Phe Glu
               100                 105                 110
   Gly Asp Asn Val Ile Leu Arg Cys Gln Gly Lys Asp Asn Lys Asn Thr
           115                 120                 125
   His Gln Lys Val Tyr Tyr Lys Asp Gly Lys Gln Leu Pro Asn Ser Tyr
       130                 135                 140
   Asn Leu Glu Lys Ile Thr Val Asn Ser Val Ser Arg Asp Asn Ser Lys
   145                 150                 155                 160
   Tyr His Cys Thr Ala Tyr Arg Lys Phe Tyr Ile Leu Asp Ile Glu Val
                   165                 170                 175
   Thr Ser Lys Pro Leu Asn Ile Gln Val Gln Glu Leu Phe Leu His Pro
               180                 185                 190
   Val Leu Arg Ala Ser Ser Ser Thr Pro Ile Glu Gly Ser Pro Met Thr
           195                 200                 205
   Leu Thr Cys Glu Thr Gln Leu Ser Pro Gln Arg Pro Asp Val Gln Leu
       210                 215                 220
   Gln Phe Ser Leu Phe Arg Asp Ser Gln Thr Leu Gly Leu Gly Trp Ser
   225                 230                 235                 240
   Arg Ser Pro Arg Leu Gln Ile Pro Ala Met Trp Thr Glu Asp Ser Gly
                   245                 250                 255
   Ser Tyr Trp Cys Glu Val Glu Thr Val Thr His Ser Ile Lys Lys Arg
               260                 265                 270
   Ser Leu Arg Ser Gln Ile Arg Val Gln Arg Val Pro Val Ser Asn Val
           275                 280                 285
   Asn Leu Glu Ile Arg Pro Thr Gly Gly Gln Leu Ile Glu Gly Glu Asn
       290                 295                 300
   Met Val Leu Ile Cys Ser Val Ala Gln Gly Ser Gly Thr Val Thr Phe
   305                 310                 315                 320
   Ser Trp His Lys Glu Gly Arg Val Arg Ser Leu Gly Arg Lys Thr Gln
                   325                 330                 335
   Arg Ser Leu Leu Ala Glu Leu His Val Leu Thr Val Lys Glu Ser Asp
   340 345 350
   Ala Gly Arg Tyr Tyr Cys Ala Ala Asp Asn Val His Ser Pro Ile Leu
   355 360 365
   Ser Thr Trp Ile Arg Val Thr Val Arg Ile Pro Val Ser His Pro Val
   370 375 380
   Leu Thr Phe Arg Ala Pro Arg Ala His Thr Val Val Gly Asp Leu Leu
   385 390 395 400
   Glu Leu His Cys Glu Ser Leu Arg Gly Ser Pro Pro Ile Leu Tyr Arg
   405 410 415
   Phe Tyr His Glu Asp Val Thr Leu Gly Asn Ser Ser Ala Pro Ser Gly
   420 425 430
   Gly Gly Ala Ser Phe Asn Leu Ser Leu Thr Ala Glu His Ser Gly Asn
   435 440 445
   Tyr Ser Cys Asp Ala Asp Asn Gly Leu Gly Ala Gln His Ser His Gly
   450 455 460
   Val Ser Leu Arg Val Thr Val Pro Val Ser Arg Pro Val Leu Thr Leu
   465 470 475 480
   Arg Ala Pro Gly Ala Gln Ala Val Val Gly Asp Leu Leu Glu Leu His
   485 490 495
   Cys Glu Ser Leu Arg Gly Ser Phe Pro Ile Leu Tyr Trp Phe Tyr His
   500 505 510
   Glu Asp Asp Thr Leu Gly Asn Ile Ser Ala His Ser Gly Gly Gly Ala
   515 520 525
   Ser Phe Asn Leu Ser Leu Thr Thr Glu His Ser Gly Asn Tyr Ser Cys
   530 535 540
   Glu Ala Asp Asn Gly Leu Gly Ala Gln His Ser Lys Val Val Thr Leu
   545 550 555 560
   Asn Val Thr Gly Thr Ser Arg Asn Arg Thr Gly Leu Thr Ala Ala Gly
   565 570 575
   Ile Thr Gly Leu Val Leu Ser Ile Leu Val Leu Ala Ala Ala Ala Ala
   580 585 590
   Leu Leu His Tyr Ala Arg Ala Arg Arg Lys Pro Gly Gly Leu Ser Ala
   595 600 605
   Thr Gly Thr Ser Ser His Ser Pro Ser Glu Cys Gln Glu Pro Ser Ser
   610 615 620
   Ser Arg Pro Ser Arg Ile Asp Pro Gln Glu Pro Thr His Ser Lys Pro
   625 630 635 640
   Leu Ala Pro Met Glu Leu Glu Pro Met Tyr Ser Asn Val Asn Pro Gly
   645 650 655
   Asp Ser Asn Pro Ile Tyr Ser Gln Ile Trp Ser Ile Gln His Thr Lys
   660 665 670
   Glu Asn Ser Ala Asn Cys Pro Met Met His Gln Glu His Glu Glu Leu
   675 680 685
   Thr Val Leu Tyr Ser Glu Leu Lys Lys Thr His Pro Asp Asp Ser Ala
   690 695 700
   Gly Glu Ala Ser Ser Arg Gly Arg Ala His Glu Glu Asp Asp Glu Glu
   705 710 715 720
   Asn Tyr Glu Asn Val Pro Arg Val Leu Leu Ala Ser Asp His
   725 730
<210> 12
   <211> 93
   <212> PRT
   <213> Homo sapiens
<400> 12
<210> 13
   <211> 960
   <212> PRT
   <213> Homo sapiens
<400> 13
   Leu Leu Gly Asn Leu Lys Thr Thr Val Glu Gly Leu Val Ser Thr Asn
   1               5                   10                  15
   Ser Pro Asn Val Trp Ser Lys Tyr Gly Gly Leu Glu Arg Leu Cys Arg
               20                  25                  30
   Asp Met Gln Ser Ile Leu Tyr His Gly Leu Ile Arg Asp Gln Ala Cys
           35                  40                  45
   Arg Arg Gln Thr Asp Tyr Trp Gln Phe Val Lys Asp Ile Arg Trp Leu
       50                  55                  60
   Ser Pro His Ser Ala Leu His Val Glu Lys Phe Ile Ser Val His Glu
   65                  70                  75                  80
   Asn Asp Gln Ser Ser Ala Asp Gly Ala Ser Glu Arg Ala Val Ala Glu
                   85                  90                  95
   Leu Trp Leu Gln His Ser Leu Gln Tyr His Cys Leu Ser Ala Gln Leu
               100                 105                 110
   Arg Pro Leu Leu Gly Asp Arg Gln Tyr Ile Arg Lys Phe Tyr Thr Asp
           115                 120                 125
   Ala Ala Phe Leu Leu Ser Asp Ala His Val Thr Ala Met Leu Gln Cys
       130                 135                 140
   Leu Glu Ala Val Glu Gln Asn Asn Pro Arg Leu Leu Ala Gln Ile Asp
   145                 150                 155                 160
   Ala Ser Met Phe Ala Arg Lys His Glu Ser Pro Leu Leu Val Thr Lys
                   165                 170                 175
   Ser Gln Ser Leu Thr Ala Leu Pro Ser Ser Thr Tyr Thr Pro Pro Asn
   180 185 190
   Ser Tyr Ala Gln His Ser Tyr Phe Gly Ser Phe Ser Ser Leu His Gln
   195 200 205
   Ser Val Pro Asn Asn Gly Ser Glu Arg Arg Ser Thr Ser Phe Pro Leu
   210 215 220
   Ser Gly Pro Pro Arg Lys Pro Gln Glu Ser Arg Gly His Val Ser Pro
   225 230 235 240
   Ala Glu Asp Gln Thr Ile Gln Ala Pro Pro Val Ser Val Ser Ala Leu
   245 250 255
   Ala Arg Asp Ser Pro Leu Thr Pro Asn Glu Met Ser Ser Ser Thr Leu
   260 265 270
   Thr Ser Pro Ile Glu Ala Ser Trp Val Ser Ser Gln Asn Asp Ser Pro
   275 280 285
   Gly Asp Ala Ser Glu Gly Pro Glu Tyr Leu Ala Ile Gly Asn Leu Asp
   290 295 300
   Pro Arg Gly Arg Thr Ala Ser Cys Gln Ser His Ser Ser Asn Ala Glu
   305 310 315 320
   Ser Ser Ser Ser Asn Leu Phe Ser Ser Ser Ser Ser Gln Lys Pro Asp
   325 330 335
   Ser Ala Ala Ser Ser Leu Gly Asp Gln Glu Gly Gly Gly Glu Ser Gln
   340 345 350
   Leu Ser Ser Val Leu Arg Arg Ser Ser Phe Ser Glu Gly Gln Thr Leu
   355 360 365
   Thr Val Thr Ser Gly Ala Lys Lys Ser His Ile Arg Ser His Ser Asp
   370 375 380
   Thr Ser Ile Ala Ser Arg Gly Ala Pro Gly Gly Pro Arg Asn Ile Thr
   385 390 395 400
   Ile Ile Val Glu Asp Pro Ile Ala Glu Ser Cys Asn Asp Lys Ala Lys
   405 410 415
   Leu Arg Gly Pro Leu Pro Tyr Ser Gly Gln Ser Ser Glu Val Ser Thr
   420 425 430
   Pro Ser Ser Leu Tyr Met Glu Tyr Glu Gly Gly Arg Tyr Leu Cys Ser
   435 440 445
   Gly Glu Gly Met Phe Arg Arg Pro Ser Glu Gly Gln Ser Leu Ile Ser
   450 455 460
   Tyr Leu Ser Glu Gln Asp Phe Gly Ser Cys Ala Asp Leu Glu Lys Glu
   465 470 475 480
   Asn Ala His Phe Ser Ile Ser Glu Ser Leu Ile Ala Ala Ile Glu Leu
   485 490 495
   Met Lys Cys Asn Met Met Ser Gln Cys Leu Glu Glu Glu Glu Val Glu
   500 505 510
   Glu Glu Asp Ser Asp Arg Glu Ile Gln Glu Leu Lys Gln Lys Ile Arg
   515 520 525
   Leu Arg Arg Gln Gln Ile Arg Thr Lys Asn Leu Leu Pro Met Tyr Gln
   530 535 540
   Glu Ala Glu His Gly Ser Phe Arg Val Thr Ser Ser Ser Ser Gln Phe
   545 550 555 560
   Ser Ser Arg Asp Ser Ala Gln Leu Ser Asp Ser Gly Ser Ala Asp Glu
   565 570 575
   Val Asp Glu Phe Glu Ile Gln Asp Ala Asp Ile Arg Arg Asn Thr Ala
   580 585 590
   Ser Ser Ser Lys Ser Phe Val Ser Ser Gln Ser Phe Ser His Cys Phe
   595 600 605
   Leu His Ser Thr Ser Ala Glu Ala Val Ala Met Gly Leu Leu Lys Gln
   610 615 620
   Phe Glu Gly Met Gln Leu Pro Ala Ala Ser Glu Leu Glu Trp Leu Val
   625 630 635 640
   Pro Glu His Asp Ala Pro Gln Lys Leu Leu Pro Ile Pro Asp Ser Leu
   645 650 655
   Pro Ile Ser Pro Asp Asp Gly Gln His Ala Asp Ile Tyr Lys Leu Arg
   660 665 670
   Ile Arg Val Arg Gly Asn Leu Glu Trp Ala Pro Pro Arg Pro Gln Ile
   675 680 685
   Ile Phe Asn Val His Pro Ala Pro Thr Arg Lys Ile Ala Val Ala Lys
   690 695 700
   Gln Asn Tyr Arg Cys Ala Gly Cys Gly Ile Arg Thr Asp Pro Asp Tyr
   705 710 715 720
   Ile Lys Arg Leu Arg Tyr Cys Glu Tyr Leu Gly Lys Tyr Phe Cys Gln
   725 730 735
   Cys Cys His Glu Asn Ala Gln Met Ala Ile Pro Ser Arg Val Leu Arg
   740 745 750
   Lys Trp Asp Phe Ser Lys Tyr Tyr Val Ser Asn Phe Ser Lys Asp Leu
   755 760 765
   Leu Ile Lys Ile Trp Asn Asp Pro Leu Phe Asn Val Gln Asp Ile Asn
   770 775 780
   Ser Ala Leu Tyr Arg Lys Val Lys Leu Leu Asn Gln Val Arg Leu Leu
   785 790 795 800
   Arg Val Gln Leu Cys His Met Lys Asn Met Phe Lys Thr Cys Arg Leu
   805 810 815
   Ala Lys Glu Leu Leu Asp Ser Phe Asp Thr Val Pro Gly His Leu Thr
   820 825 830
   Glu Asp Leu His Leu Tyr Ser Leu Asn Asp Leu Thr Ala Thr Arg Lys
   835 840 845
   Gly Glu Leu Gly Pro Arg Leu Ala Glu Leu Thr Arg Ala Gly Ala Thr
   850 855 860
   His Val Glu Arg Cys Met Leu Cys Gln Ala Lys Gly Phe Ile Cys Glu
   865 870 875 880
   Phe Cys Gln Asn Glu Asp Asp Ile Ile Phe Pro Phe Glu Leu His Lys
   885 890 895
   Cys Arg Thr Cys Glu Glu Cys Lys Ala Cys Tyr His Lys Ala Cys Phe
   900 905 910
   Lys Ser Gly Ser Cys Pro Arg Cys Glu Arg Leu Gln Ala Arg Arg Glu
   915 920 925
   Ala Leu Ala Arg Gln Ser Leu Glu Ser Tyr Leu Ser Asp Tyr Glu Glu
   930 935 940
   Glu Pro Ala Glu Ala Leu Ala Leu Glu Ala Ala Val Leu Glu Ala Thr
   945 950 955 960
<210> 14
   <211> 102
   <212> PRT
   <213> Homo sapiens
<400> 14
   Met Phe Trp Gly Gly Ile Lys Arg Gly Gly Gly Trp Gly Ala Trp Pro
   1 5 10 15
   Ser Leu Pro Gly Ser Trp Ser Phe Phe Leu Gln Gln Gly Val Leu Ala
   20 25 30
   Arg Pro Gly Pro Gly Glu Gly Ala Trp Val Gln Phe Ser Pro Ala Val
   35 40 45
   Trp Leu Ala Leu Leu Ser Ser Arg Met Gly Leu Trp Gly Leu Cys Gly
   50 55 60
   Glu Gly Arg Ser Trp Glu Pro Gly Leu Pro Val Cys Arg Leu Asp Ala
   65 70 75 80
   Ala Arg Trp Leu His Leu Gly Arg Ile Ser Arg Thr Pro Gly Leu Asp
   85 90 95
   Phe Ser Leu Asp Leu Gly
   100
<210> 15
   <211> 332
   <212> PRT
   <213> Homo sapiens
<400> 15
   Leu Ser Thr Pro Ala Ile Leu Val Thr Thr Glu Gln Val Thr Gly Phe
   1               5                   10                  15
   Ser Cys Thr Pro Trp Ala Gly Glu Arg Leu Cys Cys Ile Pro Ser Leu
               20                  25                  30
   Leu His Leu Leu Val Arg Ser Val Arg Pro Gly Pro Val Leu Gly Ala
   35 40 45
   Thr Ser Ile Leu Arg Gln Glu Ser Arg Gln Cys Cys Gln Phe His Pro
   50 55 60
   Gln Asp Ala Cys Ser Lys Pro Met His Gly Leu Ser Ser Met Pro Gly
   65 70 75 80
   Pro Gly Gly Gly Ser His Val Asp Thr Phe Thr Leu Ser Leu Leu Thr
   85 90 95
   Gln His Ile Leu Tyr Cys Ile Val Asp Ser Glu Cys Lys Ser Arg Asp
   100 105 110
   Val Leu Gln Ser Tyr Phe Asp Leu Leu Gly Glu Leu Met Lys Phe Asn
   115 120 125
   Val Asp Ala Phe Lys Arg Phe Asn Lys Tyr Ile Asn Thr Asp Ala Lys
   130 135 140
   Phe Gln Val Phe Leu Lys Gln Ile Asn Ser Ser Leu Val Asp Ser Asn
   145 150 155 160
   Met Leu Val Arg Cys Val Thr Leu Ser Leu Asp Arg Phe Glu Asn Gln
   165 170 175
   Val Asp Met Lys Val Ala Glu Val Leu Ser Glu Cys Arg Leu Leu Ala
   180 185 190
   Tyr Ile Ser Gln Val Pro Thr Gln Met Ser Phe Leu Phe Arg Leu Ile
   195 200 205
   Asn Ile Ile His Val Gln Thr Leu Thr Gln Glu Asn Val Ser Cys Leu
   210 215 220
   Asn Thr Ser Leu Val Ile Leu Met Leu Ala Arg Arg Lys Glu Arg Leu
   225 230 235 240
   Pro Leu Tyr Leu Arg Leu Leu Gln Arg Met Glu His Ser Lys Lys Tyr
   245 250 255
   Pro Gly Phe Leu Leu Asn Asn Phe His Asn Leu Leu Arg Phe Trp Gln
   260 265 270
   Gln His Tyr Leu His Lys Asp Lys Asp Ser Thr Cys Leu Glu Asn Ser
           275                     280                     285
   Ser Cys Ile Ser Phe Ser Tyr Trp Lys Glu Thr Val Ser Ile Leu Leu
       290                     295                     300
   Asn Pro Asp Arg Gln Ser Pro Ser Ala Leu Val Ser Tyr Ile Glu Glu
   305                     310                     315                     320
   Pro Tyr Met Asp Ile Asp Arg Asp Phe Thr Glu Glu
                   325                     330
<210> 16
   <211> 37
   <212> PRT
   <213> Homo sapiens
<400> 16
   Leu Ser Pro Ser Trp Cys Leu Pro Arg Ile Glu Leu Ala Thr Glu Ser
   1               5                   10                  15
   Glu Leu Ser Lys His Val Trp Ile Asp Leu Ala Asn Phe Thr Trp Phe
               20                  25                  30
   Leu Ala Val Ala Lys
           35
<210> 17
   <211> 84
   <212> PRT
   <213> Homo sapiens
<400> 17
   Met Pro Thr Pro Leu Leu Pro Leu Leu Leu Arg Leu Leu Leu Ser Cys
   1               5                   10                  15
   Leu Leu Leu Pro Ala Ala Arg Leu Ala Arg Gln Tyr Leu Leu Pro Leu
               20                  25                  30
   Leu Arg Arg Leu Ala Arg Arg Leu Gly Ser Gln Asp Met Arg Glu Ala
           35                  40                  45
   Leu Leu Gly Cys Leu Leu Phe Ile Leu Ser Gln Arg His Ser Pro Asp
       50                  55                  60
   Ala Gly Glu Ala Ser Arg Val Asp Arg Leu Glu Arg Arg Glu Arg Leu
   65                  70                  75                  80
   Gly Pro Gln Lys
<210> 18
   <211> 90
   <212> PRT
   <213> Homo sapiens
<400> 18
<210> 19
   <211> 47
   <212> PRT
   <213> Homo sapiens
<400> 19
   Met His Ile Gln Gly Ser Glu Val Leu Ala Phe Pro Ser Ala Ile Leu
   1               5                   10                  15
   Ile Cys Glu Gly Leu Leu Gly Lys Val Tyr Val Ser Pro Thr Val Pro
               20                  25                  30
   Ser Arg Gly Gly Ile Arg Ile Ser Pro Gly Gly Ser Ile Leu Leu
           35                  40                  45
<210> 20
   <211> 103
   <212> PRT
   <213> Homo sapiens
<400> 20
   Met Glu Gln Thr Ala Trp Ala Trp Arg Ala Gly Asp Gln Val Glu Ala
   1               5                   10                  15
   Ala Gln Leu Cys Leu Asn Ser Phe Ser His Arg Phe Pro Gly Arg His
               20                  25                  30
   Ser Trp Gln Pro Leu Arg Pro Met Thr Gly Thr Val Asn Ser Leu Arg
           35                  40                  45
   Gln Lys Gly Lys Asp Asn Thr Asn Val Pro Met Asn Pro Arg Asn Met
       50                  55                  60
   Lys Glu Gly His Thr Pro Thr Val Glu His Val Glu Ala Glu Cys Gly
   65                  70                  75                  80
   Ser Val Lys Gly Asp Gly Ala Pro Gly Pro Ser Met Phe Arg Ala Ala
                   85                  90                  95
   Cys Leu Cys Ser Asp Gly Arg
               100
<210> 21
   <211> 4728
   <212> DNA
   <213> Homo sapiens
<400> 21
   gtagtgaagg ggtttcccat atgaaaaata cagaaagaat tatttgaata ctagcaaata 60
   cacaacttga tatttctaga gaacccaggc acagtcttgg agacattact cctgagagac 120
   tgcagctgat ggaagatgag ccccaacttc taaaaatgta tcactaccgg gattgagata 180
   caaacagcat ttaggaaggt ctcatctgag tagcagcttc ctgccctcct tcttggagat 240
   aagtcgggct tttggtgaga cagactttcc caaccctctg cccggccggt gcccatgctt 300
   ctgtggctgc tgctgctgat cctgactcct ggaagagaac aatcaggggt ggccccaaaa 360
   gctgtacttc tcctcaatcc tccatggtcc acagccttca aaggagaaaa agtggctctc 420
   atatgcagca gcatatcaca ttccctagcc cagggagaca catattggta tcacgatgag 480
   aagttgttga aaataaaaca tgacaagatc caaattacag agcctggaaa ttaccaatgt 540
   aagacccgag gatcctccct cagtgatgcc gtgcatgtgg aattttcacc tgactggctg 600
   atcctgcagg ctttacatcc tgtctttgaa ggagacaatg tcattctgag atgtcagggg 660
   aaagacaaca aaaacactca tcaaaaggtt tactacaagg atggaaaaca gcttcctaat 720
   agttataatt tagagaagat cacagtgaat tcagtctcca gggataatag caaatatcat 780
   tgtactgctt ataggaagtt ttacatactt gacattgaag taacttcaaa acccctaaat 840
   atccaagttc aagagctgtt tctacatcct gtgctgagag ccagctcttc cacgcccata 900
   gaggggagtc ccatgaccct gacctgtgag acccagctct ctccacagag gccagatgtc 960
   cagctgcaat tctccctctt cagagatagc cagaccctcg gattgggctg gagcaggtcc 1020
   cccagactcc agatccctgc catgtggact gaagactcag ggtcttactg gtgtgaggtg 1080
   gagacagtga ctcacagcat caaaaaaagg agcctgagat ctcagatacg tgtacagaga 1140
   gtccctgtgt ctaatgtgaa tctagagatc cggcccaccg gagggcagct gattgaagga 1200
   gaaaatatgg tccttatttg ctcagtagcc cagggttcag ggactgtcac attctcctgg 1260
   cacaaagaag gaagagtaag aagcctgggt agaaagaccc agcgttccct gttggcagag 1320
   ctgcatgttc tcaccgtgaa ggagagtgat gcagggagat actactgtgc agctgataac 1380
   gttcacagcc ccatcctcag cacgtggatt cgagtcaccg tgagaattcc ggtatctcac 1440
   cctgtcctca ccttcagggc tcccagggcc cacactgtgg tgggggacct gctggagctt 1500
   cactgtgagt ccctgagagg ctctcccccg atcctgtacc gattttatca tgaggatgtc 1560
   accctgggga acagctcagc cccctctgga ggaggagcct ccttcaacct ctctctgact 1620
   gcagaacatt ctggaaacta ctcctgtgat gcagacaatg gcctgggggc ccagcacagt 1680
   catggagtga gtctcagggt cacagttccg gtgtctcgcc ccgtcctcac cctcagggct 1740
   cccggggccc aggctgtggt gggggacctg ctggagcttc actgtgagtc cctgagaggc 1800
   tccttcccga tcctgtactg gttttatcac gaggatgaca ccttggggaa catctcggcc 1860
   cactctggag gaggggcatc cttcaacctc tctctgacta cagaacattc tggaaactac 1920
   tcatgtgagg ctgacaatgg cctgggggcc cagcacagta aagtggtgac actcaatgtt 1980
   acaggaactt ccaggaacag aacaggcctt accgctgcgg gaatcacggg gctggtgctc 2040
   agcatcctcg tccttgctgc tgctgctgct ctgctgcatt acgccagggc ccgaaggaaa 2100
   ccaggaggac tttctgccac tggaacatct agtcacagtc ctagtgagtg tcaggagcct 2160
   tcctcgtcca ggccttccag gatagaccct caagagccca ctcactctaa accactagcc 2220
   ccaatggagc tggagccaat gtacagcaat gtaaatcctg gagatagcaa cccgatttat 2280
   tcccagatct ggagcatcca gcatacaaaa gaaaactcag ctaattgtcc aatgatgcat 2340
   caagagcatg aggaacttac agtcctctat tcagaactga agaagacaca cccagacgac 2400
   tctgcagggg aggctagcag cagaggcagg gcccatgaag aagatgatga agaaaactat 2460
   gagaatgtac cacgtgtatt actggcctca gaccactagc cccttaccca gagtggccca 2520
   caggaaacag cctgcaccat ttttttttct gttctctcca accacacatc atccatctct 2580
   ccagactctg cctcctacga ggctgggctg cagggtatgt gaggctgagc aaaaggtctg 2640
   caaatctccc ctgtgcctga tctgtgtgtt ccccaggaag agagcaggca gcctctgagc 2700
   aagcactgtg ttattttcac agtggagaca cgtggcaagg caggagggcc ctcagctcct 2760
   agggctgtcg aatagaggag gagagagaaa tggtctagcc agggttacaa gggcacaatc 2820
   atgaccattt gatccaagtg tgatcgaaag ctgttaatgt gctctctgta taaacaattt 2880
   gctccaaata ttttgtttcc cttttttgtg tggctggtag tggcattgct gatgttttgg 2940
   tgtatatgct gtatccttgc taccatattg ggaacagcca aaagaagtta tagaacaaga 3000
   atttaaggtg actctatctg aagtgtattt ttgtacttac agggtgacat tcccaaccaa 3060
   attaccctag ttatgatgaa aaataacttc agcatttcat taaagactct gctagtttaa 3120
   tatgtgactt gtatccccac tgcaaagacc ttatgtgtga agaatcacat taattgtaat 3180
   ttttgcttca tgacatagtc tcatcatttg ccatacatga tagatttcta gtcagtcagt 3240
   tttattctta taagcaccca ttaaccccga gacaataacc tactatatct atgtggcttc 3300
   tcccattctc ttcctctacc tcactccatc tgataaaaaa ccattctaaa tctcatgttc 3360
   attattccca tgctctccta tttctatcat attaatgtat ctacatgttt tccttaaata 3420
   tgtttttata ttagttttaa ctataagtta aagaccatat tgttgtagat aatttttttt 3480
   agtactttct cttcatgttg tatttctaag attcatccat attgttgcgt gttgctatag 3540
   ttcatttgtt tttattgctg tttagtattt acttgtgtaa aatatctggc ttaatgtttt 3600
   cctagctatc accatcaaaa actctttcca cagtgtgttg aatttttaat atgacaaaaa 3660
   tgaaaatgta ccaacaattt tcagtgactt cacctccatt ctgaaatcct gatgtttcca 3720
   aatatctctg aacacctcaa gtcctaggga caactgagat tatattaaca ttaatctctg 3780
   aatgttgcca attctaggcc ttcacttggt tcatgtagga acaccaagtc cctttcaaag 3840
   caccacatct tcctctaatc aatatttctt ggagtcccta gggaatgtct tacatgcatt 3900
   caaacaatca ccatttctgg agatacacta cagggtcacc ataaactctg ctacccttag 3960
   gttccatcac tatggaagct gagtttcacc agaaggcact tttgtcctcc attacgacca 4020
   gcaaagccag ctaagccaca gctgctggcc tcaaaaaatg tgatgatcaa tccacactgc 4080
   tcccactggc ctctgttacc cttatcctgg cctttgagtg cagggcatga tgtcctgccc 4140
   gtactgagat gctgatctct gccagttcat gttcatattg gcattaaaat tttaaggtcc 4200
   cttgaagagg gaggaggcaa atgtctctgt cttctatgtg atacattctg ctgttttttt 4260
   ctctatggtg aaaatatgta aaccggtttt gggtaccaac caccaggctg tatatggagg 4320
   ctctcctcct ttctaaccct gctgctgatt tggaattacc ttgccaagcc cctttgtgcc 4380
   ttatagtgaa ctttctctaa gggacctgtc atctcttatc attgtttatc cattttctag 4440
   attctgaacc caagaaagaa caaagttcaa gattttccat gtctttgtaa cacttagccc 4500
   tgtgcaaatc agagtatgtg agtggaagaa ggggtgagtc ctaactgtac atctcggata 4560
   taacaaatgt gcaaattctg attgattgcc ctgtaaaatg aattattctc atgcagtgct 4620
   ctacttgact tttatctttg aattcacaac taaaaaccca tagcccagaa atctaaaaaa 4680
   agtaatttta gtggagcctt tgaaaataaa agaccattgg aaaaagta 4728
<210> 22
   <211> 439
   <212> DNA
   <213> Homo sapiens
<400> 22
   ccaggagcgt catcctgcac agacacctgg ggttcatatt aaaatggaac aatgaggaat 60
   ggtcaggctt ttaagcaacc tcatctttct gctcgtttca gtctgggagt ggggggacct 120
   ttcatacaag ttgtcccata atcgtgataa aaatgtgtgt gagatatcca ctttgaagac 180
   aggggcgagg gaagttcact ttcctgaaca tacaaataat ttggcccatc cacaccaacc 240
   gtctagtgga tatcttctca acgccattac actcttctgg cccctaaaaa tactcatttt 300
   aattaataat tgccagagga aagaagaaat atttgagtaa gacatgtagt ttccctttag 360
   agtcattttt taatccagca aattgcaaag ccaggccagg cacggtggct gacacctgta 420
   atccccgcac tttgagaag 439
<210> 23
   <211> 6644
   <212> DNA
   <213> Homo sapiens
<400> 23
   ccttaactcc aaagtaaatg ctaacgtttc ccgtgtctgt accttctctc aatcggcact     60
   ctcaaaacat ctggcacggt gttcactacc ctaataagac ctcagcttcc ctcgggaggg    120
   gagggagcac tggcagttgc tgggtaattt gaagacgacg gtggagggtt tggtatcaac    180
   caacagcccc aacgtctggt ctaagtatgg tggcttggag cggctttgca gggacatgca    240
   gagcatcctc tatcacgggc ttatccgtga ccaggcgtgc cgccgccaga cggattactg    300
   gcagttcgtg aaagacatcc ggtggctcag tccccactca gcccttcacg tggagaagtt    360
   catcagcgtg cacgagaacg accagagcag tgctgatggt gccagtgaac gtgctgttgc    420
   cgagctgtgg ctgcagcaca gcctgcagta ccactgcctc tcagcccagc tccggcccct    480
   gctcggggat agacagtata tcagaaaatt ctacacagat gctgccttcc tgctaagtga    540
   cgctcatgtc acggccatgc tgcagtgcct ggaagcagtg gaacagaaca acccccgcct    600
   cctggctcag atcgatgcgt ccatgtttgc cagaaagcac gagagcccgc tcctggtgac    660
   aaagagccag agcctgacag ccctgcccag ttccacatac acccctccaa acagctatgc    720
   tcagcattcc tactttgggt ccttctctag cctccaccaa tccgtgccca acaatggctc    780
   agagagaaga tctacttcct ttccactctc tggccctccc cggaaacctc aagaaagcag    840
   agggcacgtc tcaccagcag aggatcaaac catccaagcc cccccagttt cagtctctgc    900
   actagccagg gattcccctt tgaccccaaa tgaaatgagc tccagtactc tgaccagccc    960
   catagaggca tcctgggtca gcagccagaa tgattcccca ggtgatgcca gtgaggggcc   1020
   tgagtacctg gccattggca acttggaccc ccgaggccgg actgccagct gtcagagtca   1080
   cagcagcaat gccgagagca gcagttccaa tttgttctcc tccagcagct cccagaagcc   1140
   agattctgct gcctcttcct taggggacca ggaaggaggt ggggagagcc agctgtccag   1200
   tgtcctccgc aggtccagct tctcagaggg gcagacactc actgtcacca gtggggcaaa   1260
   gaaaagccac attcgctccc attcggatac cagcattgcc tccaggggag ctccaggagg   1320
   ccccaggaat atcaccatta tagttgaaga tcccattgca gaatcctgca atgataaggc   1380
   gaagttgaga ggccctttgc cctactctgg tcaaagcagt gaagtcagca cacccagctc   1440
   tctgtacatg gaatatgaag gtggtcggta cctgtgctca ggggaaggca tgttccgaag   1500
   accatcagaa ggacagtccc tcatcagcta cctctctgag caagacttcg gcagctgtgc   1560
   cgacctggaa aaggagaatg cccacttcag catctcagag tccttaattg ctgccatcga   1620
   gctaatgaag tgcaacatga tgagccagtg cctagaggag gaggaagtgg aagaggaaga   1680
   cagtgataga gagatccagg agctgaagca gaagatccgc cttcggcgcc agcaaatccg   1740
   caccaagaac ctgctcccca tgtaccagga ggctgagcac ggaagctttc gggtcacctc   1800
   cagcagctcc cagttcagct cacgtgattc ggcacagctc tctgactctg gctctgctga   1860
   tgaggttgat gaatttgaaa tccaagatgc tgacatcaga aggaacacag cctcaagcag   1920
   caaatccttc gtttcctccc agtccttctc ccactgcttc ctgcactcca cgtctgctga   1980
   ggcggtggcc atggggctcc tgaagcagtt tgaggggatg cagcttccag ccgcctcgga   2040
   gctggagtgg cttgtcccgg agcatgatgc ccctcagaag ctcctgccca ttcctgactc   2100
   actgcccatc tcaccggatg acgggcagca cgctgacatc tacaagctgc ggattcgtgt   2160
   tcgtggcaac ttggagtggg ccccgccccg gcctcagata atttttaatg ttcatccagc   2220
   cccaacgagg aaaattgccg tggccaagca gaattaccgc tgtgcaggat gtggcatccg   2280
   gactgaccct gattacatca agcgactgcg gtactgtgag tacctgggca agtacttctg   2340
   ccagtgctgc cacgagaatg cccagatggc catccccagc cgggttctgc gcaagtggga   2400
   cttcagcaag tactacgtca gcaacttctc caaggacctg ctcattaaga tctggaatga   2460
   tcctctcttc aacgtgcagg acataaacag tgccctctat aggaaggtca agctgctcaa 2520
   tcaagtccgg ctgctgcggg tccagctgtg tcacatgaag aacatgttca agacttgccg 2580
   actggccaag gagcttctgg attcctttga cacagtccca ggccacctga cagaggacct 2640
   ccacctgtac tcactgaatg acctgactgc gaccaggaag ggggagctgg ggccccggct 2700
   tgctgagctc accagggcag gggctaccca tgtggagaga tgcatgctct gccaagccaa 2760
   aggcttcatc tgtgagttct gtcagaatga ggatgacatc atctttccct ttgagctcca 2820
   taagtgccgg acctgtgaag agtgtaaagc gtgttaccat aaagcctgct tcaagtctgg 2880
   aagctgtccg cgctgcgagc ggctgcaggc ccggcgggag gcactggcca ggcagagcct 2940
   ggagtcttac ctgtcagact acgaggagga gcccgcggaa gcgctggccc tggaagccgc 3000
   cgtcctggag gccacctgaa gaaagcacgt gcagccctcc ctccgggccg ggtcacacct 3060
   gttgcagaac tgagccactc tttgaaggac tcgccccacc tggggcttct tttttttttt 3120
   ttttttttaa ttatcatcat cttttttttt ttttttactg acttgtctga cgtctgtgtg 3180
   cagtcagccg tcggcaggtt gatgggtcca gagtctgtgg tgacagataa tttgtaaaca 3240
   ccaggtgttt ccatcagaac tgacatgcgg gtccttcagt gaagcttcta gtgcctctgt 3300
   cagtggaaga gacagcaaga ccaagttctt ccagcgtctg tggccttctc ctctaggttt 3360
   cacctgcatg tcaggtatca tttccaattt tcctttgttt cagttctgga gcttctgagc 3420
   caggcctttc tcaaccacct ctcctgctgc tgaaacgggg atggcgtttt ccctctccct 3480
   gtcctggact ggggtcagac tgtgccccga ggagaagcag cagagaatag gactacgtca 3540
   tgggcatttc gtccacttat ttgggtattt tgggggccac agaacaatcc tgactatcct 3600
   agactcctca gagacctcag aggcagctgt gaatgtccct atgttgccgg gagttcctgt 3660
   ttgaaatatt tgaagcatag aggatgccac aagctgactt tcttcatcta ccttggtgat 3720
   cttgaagcaa agaacagaac tgatgctcag gccaggctca cctgtagcct tacgccgcaa 3780
   gcatacgtga ggcgccagct ctgtcgctga aggagcgctt actcagagga gcggtcggcc 3840
   ccctcttggt gttaaggtct cttagttaac ctggcttttt ggtgcaggtg tgatctttga 3900
   agctcaggca ggtccctgat gccatcctaa ggtgaggaca ggaacctcac ccaccatctt 3960
   cttagcgtgt ccctgatgac tctgtcctct gttagatggt cgttgtgctt ctgagtaaaa 4020
   gtacaacccg actccgttct ctccccttcc tgcagcagag ctgggtcctt ccctggtggc 4080
   cgagtctctc ttgccttagc ttctttggtc aaagttggag aaaagcttcc tgctattagt 4140
   gctgttacag aacttgacgg tttgtggatg tgagtgtgaa tgtccctgtg ttcttgggat 4200
   aacaagagcc tttatgccaa ttatgcactt aactctgtgt agcctggtaa tgtttatctg 4260
   ttcatttgat aatgctgatt ttagtgtgct gcccccctcc ccccgttaat gtgtgttgat 4320
   ggtgaagtcc ttttgataat gctgattttg gtgtgctgcc tcccccttcc cccccgttaa 4380
   tgtgtgtgtt gacagtgaag tccttgggtg gggccatgtg tgtgtttgtg atgttcctta 4440
   agttgatgca gcttctaacc tctgtgaaaa cactggtcag agtggcttct ccaagagctg 4500
   gcagctctgt gaactaaagc ctgcatcatt tttgttctgg gattgaattc tgcccatggg 4560
   catgtcttct catagttgct tgctggtagg aaagaaatgg gcgtgggtgc tgccctggaa 4620
   gctgagcgga aagttgcctg tggttggtgg aagctgatga gagcttgagc tggcggtaag 4680
   aaggagtctc ccagggaagt gggagaggca ttaaggtgat ggccagggct gaggctccac    4740
   cagcgtgaga gggaacatgt gggaactggc ccctgccctt gattcctctg cctcaaagtt    4800
   gggatctgaa agccatgtag ggctagaaga ccctgaggct gttctccctt ctgttcatag 4860
   tgagactcaa aaagccaagt cccagaagtt ctgaagggct gtgactagaa gtgcccaggt 4920
   ccttcaggga gctttaagaa tgaccccaca gaactcaagt ttaactaggg gttaggtccc 4980
   agattcagac ccaggagttt ataaaaatga gctctacttc cagttttggt ttaaattaca 5040
   catccaggcc aggcacagtg gctcacacct gtaatcccag cactttggga ggccagtgcg 5100
   ggcggatcat gaggtcagga gtttgagacc agcctggcca atgtggtgaa accctgtctc 5160
   ttccaaaaat acaaaaatta gctgggcgtg gtggcacacg cctgtaatcc cagctacttg 5220
   ggaggctgag gcaggagaat cgcttgaacc tgggaggcag aggttgcagt gagccgagat 5280
   tgcgccaccg cactccagcc tgggtgacag agtgagactc cgtctcaaaa aacaaaaagg 5340
   tgacacatcc agctctttct ccaggtcact gcgctggagg acagatgtgc cgtcttgtcc 5400
   tgcctgtttc acatcagcat aggatcaaag gatgacaatg ctgacagctt ctgaagccga 5460
   actcaacagt ctcataggct cctcacttgt cacttatttt tccctagctc cctcaaccgc 5520
   accccatccc tttagatcgt gcgtctgttt tagtgactct gacacgatgc cgtcctcacc 5580
   ttccaaatac ccagttattt attcaagagg ggggaagtgg gtagaggatg ggatgttttg 5640
   gaagcacttt gcaagttacc actatctgaa aatcccctgc tgttgcgggg agaagctttg 5700
   aatgcactga agagaattcc ttctaaatga aggcaggtga tagtgttctt tctgtaagta 5760
   aagggaaaga aaaaaaacat agtttgctta ccaggtggag acaagattca agacatagca 5820
   gaagagtgga agacaaatat tttccactta aatgaggctg tttttgacgt tctctgccaa 5880
   ggatttagag ctttcgttga actaacataa aaggagtgcg agtcttagta gagatgttcc 5940
   gtgtgtgccg cccgtgctct gaactgcgtt tccacctgct gtggtgcttg tgcagcctgg 6000
   cagttcattg tcatctttaa taaactaagg aaataaaatt ctgtctttcg tcagcctgcc 6060
   ttgactgtca agaaatggca tgtgtccact gtaaagcagg tatatctgaa cattgagaag 6120
   gtttctgtgt tataatgtaa agcaaactgg accacacaca tcaaggaaag tgaggtggag 6180
   tggctgtgtc tctaacttgc catgagacct cgagcgaacc acttcacttc tctgggtttc 6240
   tggtgcatgg tgtaactgtc acttaaaatg aggtgtcttc ctctccgcaa catctgactt 6300
   gccttagatg cttcagttcc cacttctgtg agcagctagc atctttctct tctgctcctg 6360
   gcccactgga gctccagctc tacgctgcaa ctctctcaac atttcccctt aaatgtcccc 6420
   ttgtcatctc caaccagtgt gtctaaaacc agcataaatt ccgtatttcc aaaaacctaa 6480
   ccattagtca cccaggtttg aaaattaatt gtcttcaact tcctctgtaa gtggcacact 6540
   gagctcatca cgcatcgtct cattcctact ccctgtcatc aagttctgtg gatgttttcc 6600
   tctaaaataa atctctcatt acttttctgc cactagcaca caaa 6644
<210> 24
   <211> 9876
   <212> DNA
   <213> Homo sapiens
<400> 24
<210> 25
   <211> 1809
   <212> DNA
   <213> Homo sapiens
<400> 25
   caactgtggg ttccccatgg gtgcttacgg ggtgacaggt ttagagggtg gccaggccat 60
   gcaggagggt gacagtttgg ttacccagca aggcattttg accctgaaga tattttgtac 120
   taaaagttaa ttttccttta ttgaataatg gtcttcagaa aaagtaaaac ttagagcaga 180
   atggccaaag ttataattgg tctttcagat tttttcatat ggacaagaaa ctgacccacg 240
   aattataaaa tccatgtgga aaagaattga tccaaatcaa tgtaacttca agaaaatgta 300
   gaaaacttta taaaggagta aattggcttt attctcttga tgaaaactca gtattttggt 360
   gtaaactcta tttaaacaat ttcgttcata aacacaaaga caaaccatgg ggtcaaaatg 420
   tgtcctttgc ttttaaattc tgtccttcat ttacttgaat gacctcagtg cttaggcagt 480
   ggcctgtgtt ttagacctgg tgatgacagc tcccctcacc taggagctga gcaccccggc 540
   catcttggtg accacagagc aggtcacagg cttcagctgt acgccctggg caggggagag 600
   attgtgctgc attcccagtc tgctccacct cctggtgagg tctgtcaggc ctggtcctgt 660
   ccttggagcc accagcatcc tcagacaaga atctagacag tgttgccagt tccatcccca 720
   ggatgcttgc tcaaagccaa tgcatggtct gagctccatg ccaggccctg gtgggggcag 780
   ccacgttgac accttcaccc tgtccctcct cacccagcac atcctttact gcattgtgga 840
   cagcgagtgt aagtcaaggg atgtgctcca gagttacttt gacctcctgg gggagctgat 900
   gaagttcaac gttgatgcat tcaagagatt caataaatat atcaacaccg atgcaaagtt 960
   ccaggtattc ctgaagcaga tcaacagctc cctggtggac tccaacatgc tggtgcgctg 1020
   tgtcactctg tccctggacc gatttgaaaa ccaggtggat atgaaagttg ccgaggtact 1080
   gtctgaatgc cgcctgctcg cctacatatc ccaggtgccc acgcagatgt ccttcctctt 1140
   ccgcctcatc aacatcatcc acgtgcagac gctgacccag gagaacgtca gctgcctcaa 1200
   caccagcctg gtgatcctga tgctggcccg acggaaagag cggctgcccc tgtacctgcg 1260
   gctgctgcag cggatggagc acagcaagaa gtaccccggc ttcctgctca acaacttcca 1320
   caacctgctg cgcttctggc agcagcacta cctgcacaag gacaaggaca gcacctgcct 1380
   agagaacagc tcctgcatca gcttctcata ctggaaggag acagtgtcca tcctgttgaa 1440
   cccggaccgg cagtcaccct ctgctctcgt tagctacatt gaggagccct acatggacat 1500
   agacagggac ttcactgagg agtgaccttg ggccaggcct cgggaggctg ctgggccagt 1560
   gtgggtgagc gtgggtacga tgccacacgc cctgccctgt tcccgttcct ccctgctgct 1620
   ctctgcctgc cccaggtctt tgggtacagg cttggtggga gggaagtcct agaagccctt 1680
   ggtccccctg ggtctgaggg ccctaggtca tggagagcct cagtccccat aatgaggaca 1740
   gggtaccatg cccacctttc cttcagaacc ctggggccca gggccaccca gaggtaagag 1800
   gacatttag 1809
<210> 26
   <211> 876
   <212> DNA
   <213> Homo sapiens
<400> 26
   agactgggaa gtgggggcca gcccagctgc ccgctcccct caatatacca gcagttctgc 60
   ccggtgagag ctgccgtgga ttggtggggg cactgactca ctggccctgc catgcccacg 120
   ccactgctcc cgctgctgct tcgattgctg ctgtcctgcc tgctgctgcc tgctgcccgc 180
   ctggcccgcc aatacctcct gcccctgctg cgccgattgg cccgccgcct gggctcccag 240
   gacatgcgag aggctttgct gggctgtctg ctgttcattc tcagccagcg acactcgcca 300
   gacgctgggg aggcctcaag agtggaccgc ctggagagga gggagaggtt aggcccccaa 360
   aagtgaggcc acaagtcctg gcagcagctg tatccacaaa atgctttctt ttggagtagg 420
   ataatcctgg caccagcact gaccgaagcc tgcccagtgg acagaagata tagtgagggt 480
   tgtgcatgag agggatctgc cacagacatg cctctccact cccaacagaa atgtctttct 540
   ggaagaatgc cttgcatcta gcacaaaact gattattgcc cctctgtcct ccagcagttc 600
   ctcccaaaga ccactcctaa tcacctctgg cctcaggcgg gaggggaact aacacccacc 660
   cacccctgcc ctccctgcaa atgggaacat caaggttccc agtgcttaac tgagggacaa 720
   gtgacaattt agcagagagg caagatttga atccagactg tcttccagac tcaggaccta 780
   ccttaaaata atatctgagt tgcttatgga ggcagacctg cctgcaaagc ccagcactca 840
   gcaagtgctc aataaatatt tgatttgaat tctttc 876
<210> 27
   <211> 742
   <212> PRT
   <213> Homo sapiens
<400> 27
   Met Leu Leu Trp Leu Leu Leu Leu Ile Leu Thr Pro Gly Arg Glu Gln
   1               5                   10                  15
   Ser Gly Val Ala Pro Lys Ala Val Leu Leu Leu Asn Pro Pro Trp Ser
               20                  25                  30
   Thr Ala Phe Lys Gly Glu Lys Val Ala Leu Ile Cys Ser Ser Ile Ser
           35                  40                  45
   His Ser Leu Ala Gln Gly Asp Thr Tyr Trp Tyr His Asp Glu Lys Leu
       50                  55                  60
   Leu Lys Ile Lys His Asp Lys Ile Gln Ile Thr Glu Pro Gly Asn Tyr
   65                  70                  75                  80
   Gln Cys Lys Thr Arg Gly Ser Ser Leu Ser Asp Ala Val His Val Glu
   85 90 95
   Phe Ser Pro Asp Trp Leu Ile Leu Gln Ala Leu His Pro Val Phe Glu
   100 105 110
   Gly Asp Asn Val Ile Leu Arg Cys Gln Gly Lys Asp Asn Lys Asn Thr
   115 120 125
   His Gln Lys Val Tyr Tyr Lys Asp Gly Lys Gln Leu Pro Asn Ser Tyr
   130 135 140
   Asn Leu Glu Lys Ile Thr Val Asn Ser Val Ser Arg Asp Asn Ser Lys
   145 150 155 160
   Tyr His Cys Thr Ala Tyr Arg Lys Phe Tyr Ile Leu Asp Ile Glu Val
   165 170 175
   Thr Ser Lys Pro Leu Asn Ile Gln Val Gln Glu Leu Phe Leu His Pro
   180 185 190
   Val Leu Arg Ala Ser Ser Ser Thr Pro Ile Glu Gly Ser Pro Met Thr
   195 200 205
   Leu Thr Cys Glu Thr Gln Leu Ser Pro Gln Arg Pro Asp Val Gln Leu
   210 215 220
   Gln Phe Ser Leu Phe Arg Asp Ser Gln Thr Leu Gly Leu Gly Trp Ser
   225 230 235 240
   Arg Ser Pro Arg Leu Gln Ile Pro Ala Met Trp Thr Glu Asp Ser Gly
   245 250 255
   Ser Tyr Trp Cys Glu Val Glu Thr Val Thr His Ser Ile Lys Lys Arg
   260 265 270
   Ser Leu Arg Ser Gln Ile Arg Val Gln Arg Val Pro Val Ser Asn Val
   275 280 285
   Asn Leu Glu Ile Arg Pro Thr Gly Gly Gln Leu Ile Glu Gly Glu Asn
   290 295 300
   Met Val Leu Ile Cys Ser Val Ala Gln Gly Ser Gly Thr Val Thr Phe
   305 310 315 320
   Ser Trp His Lys Glu Gly Arg Val Arg Ser Leu Gly Arg Lys Thr Gln
   325 330 335
   Arg Ser Leu Leu Ala Glu Leu His Val Leu Thr Val Lys Glu Ser Asp
   340 345 350
   Ala Gly Arg Tyr Tyr Cys Ala Ala Asp Asn Val His Ser Pro Ile Leu
   355 360 365
   Ser Thr Trp Ile Arg Val Thr Val Arg Ile Pro Val Ser His Pro Val
   370 375 380
   Leu Thr Phe Arg Ala Pro Arg Ala His Thr Val Val Gly Asp Leu Leu
   385 390 395 400
   Glu Leu His Cys Glu Ser Leu Arg Gly Ser Pro Pro Ile Leu Tyr Arg
   405 410 415
   Phe Tyr His Glu Asp Val Thr Leu Gly Asn Ser Ser Ala Pro Ser Gly
   420 425 430
   Gly Gly Ala Ser Phe Asn Leu Ser Leu Thr Ala Glu His Ser Gly Asn
   435 440 445
   Tyr Ser Cys Asp Ala Asp Asn Gly Leu Gly Ala Gln His Ser His Gly
   450 455 460
   Val Ser Leu Arg Val Thr Val Pro Val Ser Arg Pro Val Leu Thr Leu
   465 470 475 480
   Arg Ala Pro Gly Ala Gln Ala Val Val Gly Asp Leu Leu Glu Leu His
   485 490 495
   Cys Glu Ser Leu Arg Gly Ser Phe Pro Ile Leu Tyr Trp Phe Tyr His
   500 505 510
   Glu Asp Asp Thr Leu Gly Asn Ile Ser Ala His Ser Gly Gly Gly Ala
   515 520 525
   Ser Phe Asn Leu Ser Leu Thr Thr Glu His Ser Gly Asn Tyr Ser Cys
   530 535 540
   Glu Ala Asp Asn Gly Leu Gly Ala Gln His Ser Lys Val Val Thr Leu
   545 550 555 560
   Asn Val Thr Gly Thr Ser Arg Asn Arg Thr Gly Leu Thr Ala Ala Gly
   565 570 575
   Ile Thr Gly Leu Val Leu Ser Ile Leu Val Leu Ala Ala Ala Ala Ala
   580 585 590
   Leu Leu His Tyr Ala Arg Ala Arg Arg Lys Pro Gly Gly Leu Ser Ala
   595 600 605
   Thr Gly Thr Ser Ser His Ser Pro Ser Glu Cys Gln Glu Pro Ser Ser
   610 615 620
   Ser Arg Pro Ser Arg Ile Asp Pro Gln Glu Pro Thr His Ser Lys Pro
   625 630 635 640
   Leu Ala Pro Met Glu Leu Glu Pro Met Tyr Ser Asn Val Asn Pro Gly
   645 650 655
   Asp Ser Asn Pro Ile Tyr Ser Gln Ile Trp Ser Ile Gln His Thr Lys
   660 665 670
   Glu Asn Ser Ala Asn Cys Pro Met Met His Gln Glu His Glu Glu Leu
   675 680 685
   Thr Val Leu Tyr Ser Glu Leu Lys Lys Thr His Pro Asp Asp Ser Ala
   690 695 700
   Gly Glu Ala Ser Ser Arg Gly Arg Ala His Glu Glu Asp Asp Glu Glu
   705 710 715 720
   Asn Tyr Glu Asn Ile Leu Asn Pro Arg Lys Asn Lys Val Gln Asp Phe
   725 730 735
   Pro Cys Leu Cys Asn Thr
   740
<210> 28
   <211> 740
   <212> PRT
   <213> Homo sapiens
<400> 28
   Met Leu Leu Trp Leu Leu Leu Leu Ile Leu Thr Pro Gly Arg Glu Gln
   1 5 10 15
   Ser Gly Val Ala Pro Lys Ala Val Leu Leu Leu Asn Pro Pro Trp Ser
   20 25 30
   Thr Ala Phe Lys Gly Glu Lys Val Ala Leu Ile Cys Ser Ser Ile Ser
   35 40 45
   His Ser Leu Ala Gln Gly Asp Thr Tyr Trp Tyr His Asp Glu Lys Leu
   50 55 60
   Leu Lys Ile Lys His Asp Lys Ile Gln Ile Thr Glu Pro Gly Asn Tyr
   65 70 75 80
   Gln Cys Lys Thr Arg Gly Ser Ser Leu Ser Asp Ala Val His Val Glu
   85 90 95
   Phe Ser Pro Asp Trp Leu Ile Leu Gln Ala Leu His Pro Val Phe Glu
   100 105 110
   Gly Asp Asn Val Ile Leu Arg Cys Gln Gly Lys Asp Asn Lys Asn Thr
   115 120 125
   His Gln Lys Val Tyr Tyr Lys Asp Gly Lys Gln Leu Pro Asn Ser Tyr
   130 135 140
   Asn Leu Glu Lys Ile Thr Val Asn Ser Val Ser Arg Asp Asn Ser Lys
   145 150 155 160
   Tyr His Cys Thr Ala Tyr Arg Lys Phe Tyr Ile Leu Asp Ile Glu Val
   165 170 175
   Thr Ser Lys Pro Leu Asn Ile Gln Val Gln Glu Leu Phe Leu His Pro
   180 185 190
   Val Leu Arg Ala Ser Ser Ser Thr Pro Ile Glu Gly Ser Pro Met Thr
   195 200 205
   Leu Thr Cys Glu Thr Gln Leu Ser Pro Gln Arg Pro Asp Val Gln Leu
   210 215 220
   Gln Phe Ser Leu Phe Arg Asp Ser Gln Thr Leu Gly Leu Gly Trp Ser
   225 230 235 240
   Arg Ser Pro Arg Leu Gln Ile Pro Ala Met Trp Thr Glu Asp Ser Gly
   245 250 255
   Ser Tyr Trp Cys Glu Val Glu Thr Val Thr His Ser Ile Lys Lys Arg
   260 265 270
   Ser Leu Arg Ser Gln Ile Arg Val Gln Arg Val Pro Val Ser Asn Val
   275 280 285
   Asn Leu Glu Ile Arg Pro Thr Gly Gly Gln Leu Ile Glu Gly Glu Asn
   290 295 300
   Met Val Leu Ile Cys Ser Val Ala Gln Gly Ser Gly Thr Val Thr Phe
   305 310 315 320
   Ser Trp His Lys Glu Gly Arg Val Arg Ser Leu Gly Arg Lys Thr Gln
   325 330 335
   Arg Ser Leu Leu Ala Glu Leu His Val Leu Thr Val Lys Glu Ser Asp
   340 345 350
   Ala Gly Arg Tyr Tyr Cys Ala Ala Asp Asn Val His Ser Pro Ile Leu
   355 360 365
   Ser Thr Trp Ile Arg Val Thr Val Arg Thr Leu Leu Ser Pro Ser Val
   370 375 380
   Pro Val Ser His Pro Val Leu Thr Phe Arg Ala Pro Arg Ala His Thr
   385 390 395 400
   Val Val Gly Asp Leu Leu Glu Leu His Cys Glu Ser Leu Arg Gly Ser
   405 410 415
   Pro Pro Ile Leu Tyr Arg Phe Tyr His Glu Asp Val Thr Leu Gly Asn
   420 425 430
   Ser Ser Ala Pro Ser Gly Gly Gly Ala Ser Phe Asn Leu Ser Leu Thr
   435 440 445
   Ala Glu His Ser Gly Asn Tyr Ser Cys Asp Ala Asp Asn Gly Leu Gly
   450 455 460
   Ala Gln His Ser His Gly Val Ser Leu Arg Val Thr Val Pro Val Ser
   465 470 475 480
   Arg Pro Val Leu Thr Leu Arg Ala Pro Gly Ala Gln Ala Val Val Gly
   485 490 495
   Asp Leu Leu Glu Leu His Cys Glu Ser Leu Arg Gly Ser Phe Pro Ile
   500 505 510
   Leu Tyr Trp Phe Tyr His Glu Asp Asp Thr Leu Gly Asn Ile Ser Ala
   515 520 525
   His Ser Gly Gly Gly Ala Ser Phe Asn Leu Ser Leu Thr Thr Glu His
   530 535 540
   Ser Gly Asn Tyr Ser Cys Glu Ala Asp Asn Gly Leu Gly Ala Gln His
   545 550 555 560
   Ser Lys Val Val Thr Leu Asn Val Thr Gly Thr Ser Arg Asn Arg Thr
   565 570 575
   Gly Leu Thr Ala Ala Gly Ile Thr Gly Leu Val Leu Ser Ile Leu Val
   580 585 590
   Leu Ala Ala Ala Ala Ala Leu Leu His Tyr Ala Arg Ala Arg Arg Lys
   595 600 605
   Pro Gly Gly Leu Ser Ala Thr Gly Thr Ser Ser His Ser Pro Ser Glu
   610 615 620
   Cys Gln Glu Pro Ser Ser Ser Arg Pro Ser Arg Ile Asp Pro Gln Glu
   625 630 635 640
   Pro Thr His Ser Lys Pro Leu Ala Pro Met Glu Leu Glu Pro Met Tyr
   645 650 655
   Ser Asn Val Asn Pro Gly Asp Ser Asn Pro Ile Tyr Ser Gln Ile Trp
   660 665 670
   Ser Ile Gln His Thr Lys Glu Asn Ser Ala Asn Cys Pro Met Met His
   675 680 685
   Gln Glu His Glu Glu Leu Thr Val Leu Tyr Ser Glu Leu Lys Lys Thr
   690 695 700
   His Pro Asp Asp Ser Ala Gly Glu Ala Ser Ser Arg Gly Arg Ala His
   705 710 715 720
   Glu Glu Asp Asp Glu Glu Asn Tyr Glu Asn Val Pro Arg Val Leu Leu
   725 730 735
   Ala Ser Asp His
   740
<210> 29
   <211> 734
   <212> PRT
   <213> Homo sapiens
<400> 29
   Met Leu Leu Trp Leu Leu Leu Leu Ile Leu Thr Pro Gly Arg Glu Gln
   1               5                   10                  15
   Ser Gly Val Ala Pro Lys Ala Val Leu Leu Leu Asn Pro Pro Trp Ser
               20                  25                  30
   Thr Ala Phe Lys Gly Glu Lys Val Ala Leu Ile Cys Ser Ser Ile Ser
           35                  40                  45
   His Ser Leu Ala Gln Gly Asp Thr Tyr Trp Tyr His Asp Glu Lys Leu
       50                  55                  60
   Leu Lys Ile Lys His Asp Lys Ile Gln Ile Thr Glu Pro Gly Asn Tyr
   65                  70                  75                  80
   Gln Cys Lys Thr Arg Gly Ser Ser Leu Ser Asp Ala Val His Val Glu
                   85                  90                  95
   Phe Ser Pro Asp Trp Leu Ile Leu Gln Ala Leu His Pro Val Phe Glu
               100                 105                 110
   Gly Asp Asn Val Ile Leu Arg Cys Gln Gly Lys Asp Asn Lys Asn Thr
           115                 120                 125
   His Gln Lys Val Tyr Tyr Lys Asp Gly Lys Gln Leu Pro Asn Ser Tyr
       130                 135                 140
   Asn Leu Glu Lys Ile Thr Val Asn Ser Val Ser Arg Asp Asn Ser Lys
   145                 150                 155                 160
   Tyr His Cys Thr Ala Tyr Arg Lys Phe Tyr Ile Leu Asp Ile Glu Val
                   165                 170                 175
   Thr Ser Lys Pro Leu Asn Ile Gln Val Gln Glu Leu Phe Leu His Pro
               180                 185                 190
   Val Leu Arg Ala Ser Ser Ser Thr Pro Ile Glu Gly Ser Pro Met Thr
           195                 200                 205
   Leu Thr Cys Glu Thr Gln Leu Ser Pro Gln Arg Pro Asp Val Gln Leu
       210                 215                 220
   Gln Phe Ser Leu Phe Arg Asp Ser Gln Thr Leu Gly Leu Gly Trp Ser
   225 230 235 240
   Arg Ser Pro Arg Leu Gln Ile Pro Ala Met Trp Thr Glu Asp Ser Gly
   245 250 255
   Ser Tyr Trp Cys Glu Val Glu Thr Val Thr His Ser Ile Lys Lys Arg
   260 265 270
   Ser Leu Arg Ser Gln Ile Arg Val Gln Arg Val Pro Val Ser Asn Val
   275 280 285
   Asn Leu Glu Ile Arg Pro Thr Gly Gly Gln Leu Ile Glu Gly Glu Asn
   290 295 300
   Met Val Leu Ile Cys Ser Val Ala Gln Gly Ser Gly Thr Val Thr Phe
   305 310 315 320
   Ser Trp His Lys Glu Gly Arg Val Arg Ser Leu Gly Arg Lys Thr Gln
   325 330 335
   Arg Ser Leu Leu Ala Glu Leu His Val Leu Thr Val Lys Glu Ser Asp
   340 345 350
   Ala Gly Arg Tyr Tyr Cys Ala Ala Asp Asn Val His Ser Pro Ile Leu
   355 360 365
   Ser Thr Trp Ile Arg Val Thr Val Arg Ile Pro Val Ser His Pro Val
   370 375 380
   Leu Thr Phe Arg Ala Pro Arg Ala His Thr Val Val Gly Asp Leu Leu
   385 390 395 400
   Glu Leu His Cys Glu Ser Leu Arg Gly Ser Pro Pro Ile Leu Tyr Arg
   405 410 415
   Phe Tyr His Glu Asp Val Thr Leu Gly Asn Ser Ser Ala Pro Ser Gly
   420 425 430
   Gly Gly Ala Ser Phe Asn Leu Ser Leu Thr Ala Glu His Ser Gly Asn
   435 440 445
   Tyr Ser Cys Asp Ala Asp Asn Gly Leu Gly Ala Gln His Ser His Gly
   450 455 460
   Val Ser Leu Arg Val Thr Val Pro Val Ser Arg Pro Val Leu Thr Leu
   465                 470                 475                 480
   Arg Ala Pro Gly Ala Gln Ala Val Val Gly Asp Leu Leu Glu Leu His
                   485                 490                 495
   Cys Glu Ser Leu Arg Gly Ser Phe Pro Ile Leu Tyr Trp Phe Tyr His
               500                 505                 510
   Glu Asp Asp Thr Leu Gly Asn Ile Ser Ala His Ser Gly Gly Gly Ala
           515                 520                 525
   Ser Phe Asn Leu Ser Leu Thr Thr Glu His Ser Gly Asn Tyr Ser Cys
       530                 535                 540
   Glu Ala Asp Asn Gly Leu Gly Ala Gln His Ser Lys Val Val Thr Leu
   545                 550                 555                 560
   Asn Val Thr Gly Thr Ser Arg Asn Arg Thr Gly Leu Thr Ala Ala Gly
                   565                 570                 575
   Ile Thr Gly Leu Val Leu Ser Ile Leu Val Leu Ala Ala Ala Ala Ala
               580                 585                 590
   Leu Leu His Tyr Ala Arg Ala Arg Arg Lys Pro Gly Gly Leu Ser Ala
           595                 600                 605
   Thr Gly Thr Ser Ser His Ser Pro Ser Glu Cys Gln Glu Pro Ser Ser
       610                 615                 620
   Ser Arg Pro Ser Arg Ile Asp Pro Gln Glu Pro Thr His Ser Lys Pro
   625                 630                 635                 640
   Leu Ala Pro Met Glu Leu Glu Pro Met Tyr Ser Asn Val Asn Pro Gly
                   645                 650                 655
   Asp Ser Asn Pro Ile Tyr Ser Gln Ile Trp Ser Ile Gln His Thr Lys
               660                 665                 670
   Glu Asn Ser Ala Asn Cys Pro Met Met His Gln Glu His Glu Glu Leu
           675                 680                 685
   Thr Val Leu Tyr Ser Glu Leu Lys Lys Thr His Pro Asp Asp Ser Ala
       690                 695                 700
   Gly Glu Ala Ser Ser Arg Gly Arg Ala His Glu Glu Asp Asp Glu Glu
   705 710 715 720
   Asn Tyr Glu Asn Val Pro Arg Val Leu Leu Ala Ser Asp His
   725 730
<210> 30
   <211> 823
   <212> PRT
   <213> Homo sapiens
<220>
   <221> misc_feature
   <222> (697)..(797)
   <223> Xaa can be any naturally occurring amino acid
<400> 30
   Met Leu Leu Trp Leu Leu Leu Leu Ile Leu Thr Pro Gly Arg Glu Gln
   1 5 10 15
   Ser Gly Val Ala Pro Lys Ala Val Leu Leu Leu Asn Pro Pro Trp Ser
   20 25 30
   Thr Ala Phe Lys Gly Glu Lys Val Ala Leu Ile Cys Ser Ser Ile Ser
   35 40 45
   His Ser Leu Ala Gln Gly Asp Thr Tyr Trp Tyr His Asp Glu Lys Leu
   50 55 60
   Leu Lys Ile Lys His Asp Lys Ile Gln Ile Thr Glu Pro Gly Asn Tyr
   65 70 75 80
   Gln Cys Lys Thr Arg Gly Ser Ser Leu Ser Asp Ala Val His Val Glu
   85 90 95
   Phe Ser Pro Asp Trp Leu Ile Leu Gln Ala Leu His Pro Val Phe Glu
   100 105 110
   Gly Asp Asn Val Ile Leu Arg Cys Gln Gly Lys Asp Asn Lys Asn Thr
   115 120 125
   His Gln Lys Val Tyr Tyr Lys Asp Gly Lys Gln Leu Pro Asn Ser Tyr
   130 135 140
   Asn Leu Glu Lys Ile Thr Val Asn Ser Val Ser Arg Asp Asn Ser Lys
   145 150 155 160
   Tyr His Cys Thr Ala Tyr Arg Lys Phe Tyr Ile Leu Asp Ile Glu Val
   165 170 175
   Thr Ser Lys Pro Leu Asn Ile Gln Val Gln Glu Leu Phe Leu His Pro
   180 185 190
   Val Leu Arg Ala Ser Ser Ser Thr Pro Ile Glu Gly Ser Pro Met Thr
   195 200 205
   Leu Thr Cys Glu Thr Gln Leu Ser Pro Gln Arg Pro Asp Val Gln Leu
   210 215 220
   Gln Phe Ser Leu Phe Arg Asp Ser Gln Thr Leu Gly Leu Gly Trp Ser
   225 230 235 240
   Arg Ser Pro Arg Leu Gln Ile Pro Ala Met Trp Thr Glu Asp Ser Gly
   245 250 255
   Ser Tyr Trp Cys Glu Val Glu Thr Val Thr His Ser Ile Lys Lys Arg
   260 265 270
   Ser Leu Arg Ser Gln Ile Arg Val Gln Arg Val Pro Val Ser Asn Val
   275 280 285
   Asn Leu Glu Ile Arg Pro Thr Gly Gly Gln Leu Ile Glu Gly Glu Asn
   290 295 300
   Met Val Leu Ile Cys Ser Val Ala Gln Gly Ser Gly Thr Val Thr Phe
   305 310 315 320
   Ser Trp His Lys Glu Gly Arg Val Arg Ser Leu Gly Arg Lys Thr Gln
   325 330 335
   Arg Ser Leu Leu Ala Glu Leu His Val Leu Thr Val Lys Glu Ser Asp
   340 345 350
   Ala Gly Arg Tyr Tyr Cys Ala Ala Asp Asn Val His Ser Pro Ile Leu
   355 360 365
   Ser Thr Trp Ile Arg Val Thr Val Arg Ile Pro Val Ser His Pro Val
   370 375 380
   Leu Thr Phe Arg Ala Pro Arg Ala His Thr Val Val Gly Asp Leu Leu
   385 390 395 400
   Glu Leu His Cys Glu Ser Leu Arg Gly Ser Pro Pro Ile Leu Tyr Arg
   405 410 415
   Phe Tyr His Glu Asp Val Thr Leu Gly Asn Ser Ser Ala Pro Ser Gly
               420                 425                 430
   Gly Gly Ala Ser Phe Asn Leu Ser Leu Thr Ala Glu His Ser Gly Asn
           435                 440                 445
   Tyr Ser Cys Asp Ala Asp Asn Gly Leu Gly Ala Gln His Ser His Gly
       450                 455                 460
   Val Ser Leu Arg Val Thr Val Pro Val Ser Arg Pro Val Leu Thr Leu
   465                 470                 475                 480
   Arg Ala Pro Gly Ala Gln Ala Val Val Gly Asp Leu Leu Glu Leu His
                   485                 490                 495
   Cys Glu Ser Leu Arg Gly Ser Phe Pro Ile Leu Tyr Trp Phe Tyr His
               500                 505                 510
   Glu Asp Asp Thr Leu Gly Asn Ile Ser Ala His Ser Gly Gly Gly Ala
           515                 520                 525
   Ser Phe Asn Leu Ser Leu Thr Thr Glu His Ser Gly Asn Tyr Ser Cys
       530                 535                 540
   Glu Ala Asp Asn Gly Leu Gly Ala Gln His Ser Lys Val Val Thr Leu
   545                 550                 555                 560
   Asn Val Thr Gly Thr Ser Arg Asn Arg Thr Gly Leu Thr Ala Ala Gly
                   565                 570                 575
   Ile Thr Gly Leu Val Leu Ser Ile Leu Val Leu Ala Ala Ala Ala Ala
               580                 585                 590
   Leu Leu His Tyr Ala Arg Ala Arg Arg Lys Pro Gly Gly Leu Ser Ala
           595                 600                 605
   Thr Gly Thr Ser Ser His Ser Pro Ser Glu Cys Gln Glu Pro Ser Ser
       610                 615                 620
   Ser Arg Pro Ser Arg Ile Asp Pro Gln Glu Pro Thr His Ser Lys Pro
   625                 630                 635                 640
   Leu Ala Pro Met Glu Leu Glu Pro Met Tyr Ser Asn Val Asn Pro Gly
                   645                 650                 655
   Asp Ser Asn Pro Ile Tyr Ser Gln Ile Trp Ser Ile Gln His Thr Lys
               660                 665                 670
   Glu Asn Ser Ala Asn Cys Pro Met Met His Gln Glu His Glu Glu Leu
           675                 680                 685
   Thr Val Leu Tyr Ser Glu Leu Lys Xaa Xaa Xaa Xaa Xaa Xaa Xaa Xaa
       690                 695                 700
   Xaa Xaa Xaa Xaa Xaa Xaa Xaa Xaa Xaa Xaa Xaa Xaa Xaa Xaa Xaa Xaa
   705                 710                 715                 720
   Xaa Xaa Xaa Xaa Xaa Xaa Xaa Xaa Xaa Xaa Xaa Xaa Xaa Xaa Xaa Xaa
                   725                 730                 735
   Xaa Xaa Xaa Xaa Xaa Xaa Xaa Xaa Xaa Xaa Xaa Xaa Xaa Xaa Xaa Xaa
               740                 745                 750
   Xaa Xaa Xaa Xaa Xaa Xaa Xaa Xaa Xaa Xaa Xaa Xaa Xaa Xaa Xaa Xaa
           755                 760                 765
   Xaa Xaa Xaa Xaa Xaa Xaa Xaa Xaa Xaa Xaa Xaa Xaa Xaa Xaa Xaa Xaa
       770                 775                 780
   Xaa Xaa Xaa Xaa Xaa Xaa Xaa Xaa Xaa Xaa Xaa Xaa Xaa Arg Thr Lys
   785                 790                 795                 800
   Phe Lys Ile Phe His Val Phe Val Thr Leu Ser Pro Val Gln Ile Arg
                   805                 810                 815
   Val Cys Glu Trp Lys Lys Gly
               820
<210> 31
   <211> 639
   <212> PRT
   <213> Homo sapiens
<400> 31
   Met Leu Leu Trp Leu Leu Leu Leu Ile Leu Thr Pro Gly Arg Glu Gln
   1               5                   10                  15
   Ser Gly Val Ala Pro Lys Ala Val Leu Leu Leu Asn Pro Pro Trp Ser
               20                  25                  30
   Thr Ala Phe Lys Gly Glu Lys Val Ala Leu Ile Cys Ser Ser Ile Ser
   35 40 45
   His Ser Leu Ala Gln Gly Asp Thr Tyr Trp Tyr His Asp Glu Lys Leu
   50 55 60
   Leu Lys Ile Lys His Asp Lys Ile Gln Ile Thr Glu Pro Gly Asn Tyr
   65 70 75 80
   Gln Cys Lys Thr Arg Gly Ser Ser Leu Ser Asp Ala Val His Val Glu
   85 90 95
   Phe Ser Pro Asp Trp Leu Ile Leu Gln Ala Leu His Pro Val Phe Glu
   100 105 110
   Gly Asp Asn Val Ile Leu Arg Cys Gln Gly Lys Asp Asn Lys Asn Thr
   115 120 125
   His Gln Lys Val Tyr Tyr Lys Asp Gly Lys Gln Leu Pro Asn Ser Tyr
   130 135 140
   Asn Leu Glu Lys Ile Thr Val Asn Ser Val Ser Arg Asp Asn Ser Lys
   145 150 155 160
   Tyr His Cys Thr Ala Tyr Arg Lys Phe Tyr Ile Leu Asp Ile Glu Val
   165 170 175
   Thr Ser Lys Pro Leu Asn Ile Gln Val Gln Gly Val Pro Val Ser Asn
   180 185 190
   Val Asn Leu Glu Ile Arg Pro Thr Gly Gly Gln Leu Ile Glu Gly Glu
   195 200 205
   Asn Met Val Leu Ile Cys Ser Val Ala Gln Gly Ser Gly Thr Val Thr
   210 215 220
   Phe Ser Trp His Lys Glu Gly Arg Val Arg Ser Leu Gly Arg Lys Thr
   225 230 235 240
   Gln Arg Ser Leu Leu Ala Glu Leu His Val Leu Thr Val Lys Glu Ser
   245 250 255
   Asp Ala Gly Arg Tyr Tyr Cys Ala Ala Asp Asn Val His Ser Pro Ile
   260 265 270
   Leu Ser Thr Trp Ile Arg Val Thr Val Arg Ile Pro Val Ser His Pro
   275 280 285
   Val Leu Thr Phe Arg Ala Pro Arg Ala His Thr Val Val Gly Asp Leu
   290 295 300
   Leu Glu Leu His Cys Glu Ser Leu Arg Gly Ser Pro Pro Ile Leu Tyr
   305 310 315 320
   Arg Phe Tyr His Glu Asp Val Thr Leu Gly Asn Ser Ser Ala Pro Ser
   325 330 335
   Gly Gly Gly Ala Ser Phe Asn Leu Ser Leu Thr Ala Glu His Ser Gly
   340 345 350
   Asn Tyr Ser Cys Asp Ala Asp Asn Gly Leu Gly Ala Gln His Ser His
   355 360 365
   Gly Val Ser Leu Arg Val Thr Val Pro Val Ser Arg Pro Val Leu Thr
   370 375 380
   Leu Arg Ala Pro Gly Ala Gln Ala Val Val Gly Asp Leu Leu Glu Leu
   385 390 395 400
   His Cys Glu Ser Leu Arg Gly Ser Phe Pro Ile Leu Tyr Trp Phe Tyr
   405 410 415
   His Glu Asp Asp Thr Leu Gly Asn Ile Ser Ala His Ser Gly Gly Gly
   420 425 430
   Ala Ser Phe Asn Leu Ser Leu Thr Thr Glu His Ser Gly Asn Tyr Ser
   435 440 445
   Cys Glu Ala Asp Asn Gly Leu Gly Ala Gln His Ser Lys Val Val Thr
   450 455 460
   Leu Asn Val Thr Gly Thr Ser Arg Asn Arg Thr Gly Leu Thr Ala Ala
   465 470 475 480
   Gly Ile Thr Gly Leu Val Leu Ser Ile Leu Val Leu Ala Ala Ala Ala
   485 490 495
   Ala Leu Leu His Tyr Ala Arg Ala Arg Arg Lys Pro Gly Gly Leu Ser
   500 505 510
   Ala Thr Gly Thr Ser Ser His Ser Pro Ser Glu Cys Gln Glu Pro Ser
   515 520 525
   Ser Ser Arg Pro Ser Arg Ile Asp Pro Gln Glu Pro Thr His Ser Lys
   530 535 540
   Pro Leu Ala Pro Met Glu Leu Glu Pro Met Tyr Ser Asn Val Asn Pro
   545 550 555 560
   Gly Asp Ser Asn Pro Ile Tyr Ser Gln Ile Trp Ser Ile Gln His Thr
   565 570 575
   Lys Glu Asn Ser Ala Asn Cys Pro Met Met His Gln Glu His Glu Glu
   580 585 590
   Leu Thr Val Leu Tyr Ser Glu Leu Lys Lys Thr His Pro Asp Asp Ser
   595 600 605
   Ala Gly Glu Ala Ser Ser Arg Gly Arg Ala His Glu Glu Asp Asp Glu
   610 615 620
   Glu Asn Tyr Glu Asn Val Pro Arg Val Leu Leu Ala Ser Asp His
   625 630 635
<210> 32
   <211> 528
   <212> PRT
   <213> Homo sapiens
<400> 32
   Met Thr Leu Thr Cys Glu Thr Gln Leu Ser Pro Gln Arg Pro Asp Val
   1               5                   10                  15
   Gln Leu Gln Phe Ser Leu Phe Arg Asp Ser Gln Thr Leu Gly Leu Gly
               20                  25                  30
   Trp Ser Arg Ser Pro Arg Leu Gln Ile Pro Ala Met Trp Thr Glu Asp
           35                  40                  45
   Ser Gly Ser Tyr Trp Cys Glu Val Glu Thr Val Thr His Ser Ile Lys
       50                  55                  60
   Lys Arg Ser Leu Arg Ser Gln Ile Arg Val Gln Arg Val Pro Val Ser
   65                  70                  75                  80
   Asn Val Asn Leu Glu Ile Arg Pro Thr Gly Gly Gln Leu Ile Glu Gly
                   85                  90                  95
   Glu Asn Met Val Leu Ile Cys Ser Val Ala Gln Gly Ser Gly Thr Val
   100 105 110
   Thr Phe Ser Trp His Lys Glu Gly Arg Val Arg Ser Leu Gly Arg Lys
   115 120 125
   Thr Gln Arg Ser Leu Leu Ala Glu Leu His Val Leu Thr Val Lys Glu
   130 135 140
   Ser Asp Ala Gly Arg Tyr Tyr Cys Ala Ala Asp Asn Val His Ser Pro
   145 150 155 160
   Ile Leu Ser Thr Trp Ile Arg Val Thr Val Arg Ile Pro Val Ser His
   165 170 175
   Pro Val Leu Thr Phe Arg Ala Pro Arg Ala His Thr Val Val Gly Asp
   180 185 190
   Leu Leu Glu Leu His Cys Glu Ser Leu Arg Gly Ser Pro Pro Ile Leu
   195 200 205
   Tyr Arg Phe Tyr His Glu Asp Val Thr Leu Gly Asn Ser Ser Ala Pro
   210 215 220
   Ser Gly Gly Gly Ala Ser Phe Asn Leu Ser Leu Thr Ala Glu His Ser
   225 230 235 240
   Gly Asn Tyr Ser Cys Asp Ala Asp Asn Gly Leu Gly Ala Gln His Ser
   245 250 255
   His Gly Val Ser Leu Arg Val Thr Val Pro Val Ser Arg Pro Val Leu
   260 265 270
   Thr Leu Arg Ala Pro Gly Ala Gln Ala Val Val Gly Asp Leu Leu Glu
   275 280 285
   Leu His Cys Glu Ser Leu Arg Gly Ser Phe Pro Ile Leu Tyr Trp Phe
   290 295 300
   Tyr His Glu Asp Asp Thr Leu Gly Asn Ile Ser Ala His Ser Gly Gly
   305 310 315 320
   Gly Ala Ser Phe Asn Leu Ser Leu Thr Thr Glu His Ser Gly Asn Tyr
   325 330 335
   Ser Cys Glu Ala Asp Asn Gly Leu Gly Ala Gln His Ser Lys Val Val
   340 345 350
   Thr Leu Asn Val Thr Gly Thr Ser Arg Asn Arg Thr Gly Leu Thr Ala
   355 360 365
   Ala Gly Ile Thr Gly Leu Val Leu Ser Ile Leu Val Leu Ala Ala Ala
   370 375 380
   Ala Ala Leu Leu His Tyr Ala Arg Ala Arg Arg Lys Pro Gly Gly Leu
   385 390 395 400
   Ser Ala Thr Gly Thr Ser Ser His Ser Pro Ser Glu Cys Gln Glu Pro
   405 410 415
   Ser Ser Ser Arg Pro Ser Arg Ile Asp Pro Gln Glu Pro Thr His Ser
   420 425 430
   Lys Pro Leu Ala Pro Met Glu Leu Glu Pro Met Tyr Ser Asn Val Asn
   435 440 445
   Pro Gly Asp Ser Asn Pro Ile Tyr Ser Gln Ile Trp Ser Ile Gln His
   450 455 460
   Thr Lys Glu Asn Ser Ala Asn Cys Pro Met Met His Gln Glu His Glu
   465 470 475 480
   Glu Leu Thr Val Leu Tyr Ser Glu Leu Lys Lys Thr His Pro Asp Asp
   485 490 495
   Ser Ala Gly Glu Ala Ser Ser Arg Gly Arg Ala His Glu Glu Asp Asp
   500 505 510
   Glu Glu Asn Tyr Glu Asn Val Pro Arg Val Leu Leu Ala Ser Asp His
   515 520 525
<210> 33
   <211> 485
   <212> PRT
   <213> Homo sapiens
<400> 33
   Met Trp Thr Glu Asp Ser Gly Ser Tyr Trp Cys Glu Val Glu Thr Val
   1 5 10 15
   Thr His Ser Ile Lys Lys Arg Ser Leu Arg Ser Gln Ile Arg Val Gln
   20 25 30
   Arg Val Pro Val Ser Asn Val Asn Leu Glu Ile Arg Pro Thr Gly Gly
           35                  40                  45
   Gln Leu Ile Glu Gly Glu Asn Met Val Leu Ile Cys Ser Val Ala Gln
       50                  55                  60
   Gly Ser Gly Thr Val Thr Phe Ser Trp His Lys Glu Gly Arg Val Arg
   65                  70                  75                  80
   Ser Leu Gly Arg Lys Thr Gln Arg Ser Leu Leu Ala Glu Leu His Val
                   85                  90                  95
   Leu Thr Val Lys Glu Ser Asp Ala Gly Arg Tyr Tyr Cys Ala Ala Asp
               100                 105                 110
   Asn Val His Ser Pro Ile Leu Ser Thr Trp Ile Arg Val Thr Val Arg
           115                 120                 125
   Ile Pro Val Ser His Pro Val Leu Thr Phe Arg Ala Pro Arg Ala His
       130                 135                 140
   Thr Val Val Gly Asp Leu Leu Glu Leu His Cys Glu Ser Leu Arg Gly
   145                 150                 155                 160
   Ser Pro Pro Ile Leu Tyr Arg Phe Tyr His Glu Asp Val Thr Leu Gly
                   165                 170                 175
   Asn Ser Ser Ala Pro Ser Gly Gly Gly Ala Ser Phe Asn Leu Ser Leu
               180                 185                 190
   Thr Ala Glu His Ser Gly Asn Tyr Ser Cys Asp Ala Asp Asn Gly Leu
           195                 200                 205
   Gly Ala Gln His Ser His Gly Val Ser Leu Arg Val Thr Val Pro Val
       210                 215                 220
   Ser Arg Pro Val Leu Thr Leu Arg Ala Pro Gly Ala Gln Ala Val Val
   225                 230                 235                 240
   Gly Asp Leu Leu Glu Leu His Cys Glu Ser Leu Arg Gly Ser Phe Pro
                   245                 250                 255
   Ile Leu Tyr Trp Phe Tyr His Glu Asp Asp Thr Leu Gly Asn Ile Ser
               260                 265                 270
   Ala His Ser Gly Gly Gly Ala Ser Phe Asn Leu Ser Leu Thr Thr Glu
               275                 280                 285
   His Ser Gly Asn Tyr Ser Cys Glu Ala Asp Asn Gly Leu Gly Ala Gln
       290                 295                 300
   His Ser Lys Val Val Thr Leu Asn Val Thr Gly Thr Ser Arg Asn Arg
   305                 310                 315                 320
   Thr Gly Leu Thr Ala Ala Gly Ile Thr Gly Leu Val Leu Ser Ile Leu
                   325                 330                 335
   Val Leu Ala Ala Ala Ala Ala Leu Leu His Tyr Ala Arg Ala Arg Arg
               340                 345                 350
   Lys Pro Gly Gly Leu Ser Ala Thr Gly Thr Ser Ser His Ser Pro Ser
           355                 360                 365
   Glu Cys Gln Glu Pro Ser Ser Ser Arg Pro Ser Arg Ile Asp Pro Gln
       370                 375                 380
   Glu Pro Thr His Ser Lys Pro Leu Ala Pro Met Glu Leu Glu Pro Met
   385                 390                 395                 400
   Tyr Ser Asn Val Asn Pro Gly Asp Ser Asn Pro Ile Tyr Ser Gln Ile
                   405                 410                 415
   Trp Ser Ile Gln His Thr Lys Glu Asn Ser Ala Asn Cys Pro Met Met
               420                 425                 430
   His Gln Glu His Glu Glu Leu Thr Val Leu Tyr Ser Glu Leu Lys Lys
           435                 440                 445
   Thr His Pro Asp Asp Ser Ala Gly Glu Ala Ser Ser Arg Gly Arg Ala
       450                 455                 460
   His Glu Glu Asp Asp Glu Glu Asn Tyr Glu Asn Val Pro Arg Val Leu
   465                 470                 475                 480
   Leu Ala Ser Asp His
                   485

## Description

### Field of the invention

This invention relates to detecting the presence of, or the risk of breast cancer.

### Background of the invention

There are over 1 million cases of breast cancer per year globally, of which around 0.5 million are in the US, 40,000 are in the UK and nearly 2,000 in Ireland. It is the leading cause of cancer deaths among women (Keen and Davidson, 2003). Although the overall incidence of the disease is increasing within the western world, wider screening and improved treatments have led to a gradual decline in the fatality rate of about 1% per year since 1991. Inheritance of susceptibility genes, such as BRCA1 and BRCA2, account for only 5% of breast cancer cases and the factors responsible for the other 95% remain obscure (Grover and Martin, 2002). In the absence of a strategy to reduce causative gents of breast cancer, early detection remains the best approach to reducing the mortality rate of this disease.

It is widely held that breast cancer initiates as the pre-malignant stage of atypical ductal hyperplasia (ADH), progresses into the pre-invasive stage of ductal carcinoma *in situ* (DCIS), and culminates in the potentially lethal stage of invasive ductal carcinoma (IDC). This linear model of breast cancer progression has been the rationale for the use of detection methods such as mammography in the hope of diagnosing and treating breast cancer at earlier clinical stages (Ma et al., 2003).

Patients diagnosed with early breast cancer have greater than a 90% 5 year relative survival rate, compared to 20% for patients diagnosed with distally metastasised breast cancer. Nonetheless, there is no definitive early-stage screening test for breast cancer; diagnosis currently being made on the results of mammography and fine needle biopsy. Mammography has its limitations, with over 80% of suspicious results being false-positives and 10-15% of women with breast cancer providing false-negative results. Often the tumour has reached a late stage in development before detection, reducing the chances of survival for the patient and increasing the cost of treatment and management for the healthcare system. More sensitive methods are required to detect small (<2 cm diameter) early stage in-situ carcinomas of the breast, to reduce patient mortality. As mammography is normally only offered to women over the age of 50, cancers in younger women may develop undetected. It has been observed that pre-menopausal cancer is more aggressive, so early detection in these cases is even more important. In addition to early detection, there remain serious problems in classifying the disease as malignant or benign, in the staging of known cancers and in differentiating between tumour types. Finally, there is a need to monitor ongoing treatment effects and to identify patients becoming resistant to particular therapies. Such detection processes are further complicated, as the mammary gland is one of the few organs that undergo striking morphological and functional changes during adult life, particularly during pregnancy, lactation and involution, potentially leading to changes in the molecular signature of the same mammary gland over time.

Diagnosis of disease is often made by the careful examination of the relative levels of a small number of biological markers. Despite recent advances, the contribution of the current biomarkers to patient care and clinical outcome is limited. This is due to their low diagnostic sensitivity and disease specificity. Some molecular biomarkers, however, are being used routinely in disease diagnosis, for example prostate specific antigen in prostate cancer screening, and new candidate markers are being discovered at an increasing rate (Pritzker, 2002). It is becoming accepted that the use of a panel of well-validated biomarkers would enhance the positive predictive value of a test and minimize false positives or false negatives (Srinivas et al., 2002). In addition, there is now growing interest in neural networks, which show the promise of combining weak but independent information from various biomarkers to produce a prognostic/predictive index that is more informative than each biomarker alone (Yousef et al., 2002). As more molecular information is being collated, diseases such as breast cancer are being sub-divided according to genetic signatures linked to patient outcome, providing valuable information for the clinician. Emerging novel technologies in molecular medicine have already demonstrated their power in discriminating between disease sub-types that are not recognisable by traditional pathological criteria (Sorlie et al., 2001) and in identifying specific genetic events involved in cancer progression (Srinivas et al., 2002). Further issues need to be addressed in parallel, relating to the efficacy of biomarkers between genders and races, thus large scale screening of a diverse population is a necessity.

In addition, the management of breast cancer could be improved by the use of new markers normally expressed only in the breast, but found elsewhere in the body as a result of the disease. Predictors of the activity of the disease would also have valuable utility in the management of the disease, especially those that predict if a ductal carcinoma *in situ* will develop into invasive ductal carcinoma.

### Summary of the invention

The present invention relates to the use of an isolated polynucleotide identified herein as SEQ ID No. 4, or its complement, or a fragment thereof of at least 15 consecutive nucleotides that hybridises to SEQ ID No.4 (or a complement thereof) under stringent hybridising conditions in an *in vitro* diagnostic assay to test for the presence of or the risk of breast cancer in a patient, wherein an increased expression of the polynucleotide indicates the pressure of or the risk of breast cancer in the patient.

Moreover, the present invention relates to the use of a polynucleotide that hybridises with or inhibits the expression of a polynucleotide as defined in claim 1, in the manufacture of a medicament for the treatment of breast cancer.

### Description of the invention

The present invention is based on the identification of genes that are differentially expressed in a patient suffering cancer, in particular, breast cancer. Identification of the individual genes or their expressed products, such as mRNA or a polypeptide, in a sample obtained from a patient indicates the presence of or the risk of cancer in the patient.

The disclosure further relates to reagents such as polypeptide sequences, useful for detecting, diagnosing, monitoring, prognosticating, preventing, imaging, treating or determining a pre-disposition to cancer.

Diagnosis can be made on the basis of the presence, absence or level of expression of the gene or gene product in the patient. As used herein, the term "gene product" refers to the mRNA or polypeptide product that results from transcription of the gene. The methods to carry out the diagnosis can involve the synthesis of cDNA from the mRNA in a test sample, amplifying as appropriate portions of the cDNA corresponding to the genes or fragments thereof and detecting each product as an indication of the presence of the disease in that tissue, or detecting translation products of the mRNAs comprising gene sequences as an indication of the presence of the disease.

The presence, absence or level of expression of the gene or gene product in the patient is detected in a sample that is isolated from the patient. In a preferred embodiment, the sample material is isolated from breast tissue, for example by biopsy.

In a preferred embodiment, a plurality of the marker sequences disclosed herein are identified, either sequentially or simultaneously, in a sample or samples obtained from a patient in order to diagnose cancer. In a preferred embodiment, two, three, four, five or more marker sequences are detected.

Useful reagents include polynucleotides or fragment(s) thereof which may be useful in diagnostic methods such as RT-PCR, PCR or hybridisation assays of mRNA extracted from biopsied tissue, blood or other test samples; or proteins which are the translation products of such mRNAs; or antibodies directed against these proteins. These assays also include methods for detecting the gene products (proteins) in light of possible post-translation modifications that can occur in the body, including interactions with molecules such as co-factors, inhibitors, activators and other proteins in the formation of sub-unit complexes.

The genes associated with cancer are characterised by the polynucleotides shown as SEQ ID No. 1 - SEQ ID No. 10. The expressed polypeptide products of the genes are identified herein by SEQ ID No. 11 - SEQ ID No. 20, respectively.

How each gene is differentially expressed in cancer is indicated in the results section for each gene, below. For those genes that show increased expression in cancer (i.e. genes that are "upregulated" in a tumour sample), an increased level of a gene product in a sample isolated from a patient is indicative of the presence of, or the risk of, cancer. For those genes that show decreased expression in cancer (i.e. genes that are "downregulated" in a tumour sample), a decreased level of a gene product in a sample isolated from a patient is indicative of the presence of, or the risk of, cancer. As used herein, the terms "upregulated" and "downregulated" preferably refer to a significant change in the level of expression compared to the control. Significant levels will be apparent to the skilled person; preferably a two-fold change in expression is observed, more preferably a four-fold expression change is observed.

For the avoidance of doubt, genes characterised by SEQ ID Nos.1-6 and 10 show increased expression in cancer and genes characterised by SEQ ID Nos. 7-9 show decreased expression in cancer.

The skilled person will understand that the terms "increased" and "decreased" refer to the amount of a gene product in a sample, compared to a "control" sample, or a known level of expression, that is indicative of a "healthy" patient that does not have, or is not predisposed to, cancer. For example, in the preferred embodiment wherein the sample is isolated from the breast tissue of a patient, the expression level in this sample is compared to a "control" sample of (normal, healthy) breast tissue or known level of expression for "healthy" breast tissue.

In an alternative embodiment, the amount of gene product in a sample can be compared to a "control" sample or known level of expression that is indicative of a "diseased" patient that is known to have, or be predisposed to, cancer.

Identification of the genes or their expressed products may be carried out by techniques known for the detection or characterisation of polynucleotides or polypeptides. For example, isolated genetic material from a patient can be probed using short oligonucleotides that hybridise specifically to the target gene. The oligonucleotide probes may be detectably labelled, for example with a fluorophore, so that upon hybridisation with the target gene, the probes can be detected. Alternatively, the gene, or parts thereof, may be amplified using the polymerase enzyme, e.g. in the polymerase chain reaction, with the products being identified, again using labelled oligonucleotides.

Diagnostic assays incorporating any of these genes, proteins or antibodies will include, but not be limited to:
Polymerase chain reaction (PCR)
Reverse transcription PCR
Real-time PCR
*In-situ* hybridisation
Southern dot blots
Immuno-histochemistry
Ribonuclease protection assay
cDNA array techniques
ELISA
Protein, antigen or antibody arrays on solid supports such as glass or ceramics.
Small interfering RNA functional assays.

All of the above techniques are well known to those in the art. Preferably, the diagnostic assay is carried out *in vitro,* outside of the body of the patient.

The present disclosure is also concerned with isolated polynucleotides that comprise the sequences identified as SEQ ID No. 1 - SEQ ID No. 10, or their complements, or fragments of each thereof that comprise at least 15 consecutive nucleotides, preferably 30 nucleotides, more preferably at least 50 nucleotides. Polynucleotides that hybridise to a polynucleotide as defined above, are also disclosed. Hybridisation will usually be carried out under stringent conditions, known to those in the art and are chosen to reduce the possibility of non-complementary hybridisation. Examples of suitable conditions are disclosed in Nucleic Acid Hybridisation. A Practical Approach (B. D. Hames and S. J. Higgins, editors IRL Press, 1985). An example of stringent hybridisation conditions is overnight incubation at 42°C in a solution comprising: 50% formamide, 5x SSC (150mM NaCl, 15mM trisodium citrate), 50mM sodium phosphate (pH7.6), 5x Denhardt's solution, 10% dextran sulphate, and 20µg/ml denatured, sheared salmon sperm DNA, followed by washing in 0.1 x SSC at about 65°C.

Isolated peptides encoded by polynucleotides comprising SEQ ID No. 1 - SEQ ID No. 10 are also disclosed. Preferably a peptide comprises any of the sequences identified herein as SEQ ID No. 11 - SEQ ID No. 20, or a fragment thereof of at least 10 amino acid residues.

Homologues of SEQ ID Nos. 1 to 20 are also disclosed. The term "homologue" refers to a sequence that is similar but not identical to one of SEQ ID Nos. 1 to 20. A homologue performs the same function as SEQ ID Nos. 1 to 20, i.e. the same biological function or the function as a cancer marker. Whether two sequences are homologous is routinely calculated using a percentage similarity or identity, terms that are well known in the art. Homologues of SEQ ID Nos. 1 to 20 preferably have 70% or greater similarity or identity at the nucleic acid or amino acid level, more preferably 80% or greater, more preferably 90% or greater, such as 95% or 99% identity or similarity at the nucleic acid or amino acid level. A number of programs are available to calculate similarity or identity; preferred programs are the BLASTn, BLASTp and 8LASTx programs, run with default parameters, available at www.ncbi.nlm.nih.gov. For example, 2 nucleotide sequences may be compared using the BLASTn program with default parameters (score = 100, word length = 11, expectation value= 11, low complexity filtering = on).

The skilled person will realise that a gene or gene product identified in a patient may differ slightly from the exact gene or product sequence provided herein, yet is still recognisable as the same gene or gene product. For example, a skilled person may identify a polynucleotide or polypeptide under investigation by a partial sequence and/or a physical characteristic, such as the molecular weight of the gene product. The gene or gene product in a patient may be an isoform of that defined herein, eg. isoforms and splice variants. The skilled person will realise that there are differences in sequences between individuals, for example single nucleotide poymorphisms.

The identification of the genes characterised by SEQ ID No. 1 - SEQ ID No. 10, also permits therapies to be developed, with each gene being a target for therapeutic molecules. For example, there are now many known molecules that have been developed for gene therapy, to target and prevent the expression of a specific gene. One particular molecule is a small interfering RNA (siRNA), which suppresses the expression of a specific target protein by stimulating the degradation of the target mRNA. Other synthetic oligonucleotides are also known which can bind to a gene of interest (or its regulatory elements) to modify expression. Peptide nucleic acids (PNAs) in association with DNA (PNA-DNA chimeras) have also been shown to exhibit strong decoy activity, to alter the expression of the gene of interest. Molecules, preferably polynucleotides, that can alter the expression level of a gene characterised by SEQ ID No. 1 - SEQ ID No. 10 are therefore useful in the treatment of cancer, preferably breast cancer.

The present disclosure also relates to antibodies raised against a peptide of any of the genes identified herein. The antibodies will usually have an affinity for the peptide of at least 10⁻⁶M, more preferably, 10⁻⁹M and most preferably at least 10⁻¹¹ M. The antibody may be of any suitable type, including monoclonal or polyclonal. Assay kits for determining the presence of the peptide antigen in a test sample are also included. In one embodiment, the assay kit comprises a container comprising an antibody that specifically binds to the antigen, wherein the antigen comprises at least one epitope encoded by a gene characterised by SEQ ID No. 1 - SEQ ID No. 10. These kits can further comprise containers with useful tools for collecting test samples, such as blood, saliva, urine and stool. Such tools include lancets and absorbent paper or cloth for collecting and stabilising blood, swabs for collecting and stabilising saliva, cups for collecting and stabilising urine and stool samples. The antibody can be attached to a solid phase, such as glass or a ceramic surface.

Detection of antibodies that bind specifically to any of the antigens in a test sample suspected of containing these antibodies may also be carried out. This detection method comprises contacting the test sample with a polypeptide, which contains at least one epitope of the gene characterised by any of SEQ ID Nos. 1-10. Contact is performed for a time and under conditions sufficient to allow antigen/antibody complexes to form. The method further entails detecting complexes, which contain any of the polypeptides. The polypeptide complex can be produced recombinantly or synthetically or be purified from natural sources.

In a separate embodiment of the disclosure, antibodies, or fragments thereof, against any of the antigens can be used for the detection of the location of the antigen in a patient for the purpose of detecting or diagnosing the disease or condition. Such antibodies can be monoclonal or polyclonal, or made by molecular biology techniques and can be labelled with a variety of detectable agents, including, but not limited to radioisotopes.

In a further embodiment of the invention, antibodies or fragments thereof, whether monoclonal or polyclonal or made by molecular biology techniques, can be used as therapeutics for the disease characterised by the expression of any of the genes herein disclosed. The antibody may be used without derivatisation, or it may be derivatised with a cytotoxic agent such as radioisotope, enzyme, toxin, drug, pro-drug or the like.

The term "antibody" refers broadly to any immunologic binding agent such as IgG, IgM, IgA, IgD and IgE. Antibody is also used to refer to any antibody-like molecule that has an antigen-binding region and includes antibody fragments such as single domain antibodies (DABS), Fv, scFv, aptamers, etc. The techniques for preparing and using various antibody-based constructs and fragments are well known in the art. Means for preparing and characterising antibodies are also well known in the art.

If desired, the cancer screening methods described herein may be readily combined with other methods in order to provide an even more reliable indication of diagnosis or prognosis, thus providing a multi-marker test.

The following examples illustrate the invention.

### Examples

A number of differentially expressed gene fragments were isolated from cDNA populations derived from matched clinical samples of breast cancer patients, using non-isotopic differential display (DDRT-PCR) or through mining of the National Cancer Institutes CGAP databases. Details of these fragments are listed in table A.

**Table A**

| Differentially expressed genes discovered through database mining or DDRT-PCR. | | | | |
|---|---|---|---|---|
| **SEQ ID Nos.** | **Fragment name** | **Route of discovery** | **Size (bp)** | **Homologue** |
| 1 and 11 | RDX-BC-8 | DDRT-PCR | 4,728 | Fc receptor like 3 |
| 2 and 12 | RDX-BC -9 | DDRT-PCR | 439 | H2C10808.1 (EC Gene prediction) |
| 3 and 13 | RDX-BC -29 | DDRT-PCR | 5,396 | D86979 |
| 4 and 14 | RDX-BC -102 | DDRT-PCR | 9,876 | FLJ25967.aAug05 |
| 5 and 15 | RDX-BC -105 | DDRT-PCR | 1,819 | Transient receptor potential cation channel, subfamily C, member 4 associated protein |
| 6 and 16 | RDX-BC -192 | DDRT-PCR | 323 | None |
| 7 and 17 | RDX-BC -220 | DDRT-PCR | 876 | H6C6674.1 (EC Gene predictions) |
| 8 and 18 | RDX-BC-GAP-6 | Database mining | 966 | Smertu (Acembly Gene Prediction)/ H20C268 (EC gene predictions) |
| 9 and 19 | RDX-BC-GAP-7 | Database mining | 1,289 | Koyna (Acembly Gene Prediction)/ H11C12538 (EC gene predictions) |
| 10 and 20 | RDX-BC-GAP-23 | Database mining | 520 | Kleysu (Acembly Gene Prediction)/ H18C6270 (EC gene predictions) |

The expression profile of these novel molecular markers, their full lengths and corresponding presumed protein sequences are detailed herein.

### Materials and methods (DDRT-PCR).

Tissue samples were obtained, with full ethical approval and informed patient consent. Differential gene expression was investigated between matched sets of normal breast and tumour tissue surgically removed from the same donor. Messenger RNA was extracted and cDNA synthesised using Dynal dT18-tagged Dynabeads and Superscript III reverse transcription protocols, respectively. Differential display reverse transcription PCR (DDRT-PCR) was employed to observe differences between gene expression profiles of these matched samples. Individual gene transcripts showing up- or down-regulation were isolated and investigated further. In addition, the National Cancer Institute Cancer Genome Anatomy Project (CGAP) was trawled for breast cancer specific sequences, then checked for specificity using the virtual Northern blot programme. Fragments discovered through database mining were added to the DDRT-PCR batch and used in expression profiling.

First described by Liang & Pardee (1992) differential display reverse transcription PCR (DDRT-PCR) uses mRNA from two or more biological samples as templates for representative cDNA synthesis by reverse transcription, with one of 3 possible anchor primers. Each of the 3 subpopulations was PCR-amplified using its respective anchor primer coupled with one of 80 arbitrary 13-mer primers. This number of primer combinations has been estimated to facilitate the representation of 96% of expressed genes in an mRNA population (Sturtevant, 2000). This population sub-division results in the reduction of the estimated 12,000-15,000 mRNAs expressed in eukaryotic cells to 100-150 transcripts on completion of second strand cDNA synthesis for each primer set. This facilitates parallel electrophoretic separation and accurate visualization of matched primer sets on a polyacrylamide gel, leading to the identification of gene fragments expressed in one tissue sample but not the other.

Identification and amplification of fragments of interest was followed by removal of false positives through direct sequencing of PCR products. On-line database interrogation determined gene novelty and found their chromosomal location. Fragments not matching known genes potentially represent novel markers for the breast cancer from which they were derived. Molecular screening of each transcript was performed by real-time PCR, using a suite of matched cDNA populations from breast cancer donors. β-action was used as a reference gene for calibrating cDNA templates, after which the expression profile of each novel marker was determined, in turn. Partial transcripts of the novel gene were then extended using 5' RACE (rapid amplification of cDNA ends), which incorporates gene-specific extension and amplification, verifiable by sequencing. Alternatively, sequences homologous to known or predicted genes were extended by extrapolation of the fragment along the appropriate chromosome of the human genome. Open reading frame primers were designed and subsequent amplicons verified by direct sequence analysis and human genome database interrogation.

Tissue specific expression was determined using gene specific primers against cDNA populations derived from a comprehensive panel of up to 22 human tissue types, as follows:

| | |
|---|---|
| Adrenal gland | pooled from 62 donors |
| Bone marrow | pooled from 7 donors |
| Brain, cerebellum | pooled from 24 donors |
| Brain, whole | pooled from one donor |
| Colon* | pooled from one donor |
| Foetal brain | pooled from 59 donors |
| Foetal liver | pooled from 63 donors |
| Heart | pooled from one donor |
| Kidney | pooled from one donor |
| Liver | pooled from one donor |
| Lung | pooled from one donor |
| Placenta | pooled from 7 donors |
| Prostate | pooled from 47 donors |
| Salivary gland | pooled from 24 donors |
| Skeletal muscle | pooled from 2 donors |
| Small intestine* | pooled from one donor |
| Spleen | pooled from 14 donors |
| Testis | pooled from 19 donors |
| Thymus | pooled from 9 donors |
| Thyroid gland | pooled from 65 donors |
| Trachea | pooled from male/female Caucasians, ages 18-54 |
| Uterus | pooled from 10 donors |

Note that the majority of these samples were part of the Human Total RNA panel II (Clontech), but two RNA samples, marked with asterisks, were obtained separately from Clontech. In addition, assays were performed on a range of human tumour samples, obtained through Medical Solutions plc, Nottingham, UK. cDNA representative of tumours from ovary, testis, stomach, liver, lung, bladder, colon and pancreas were assayed against β-actin and the putative markers, by real-time PCR.

In conjunction with novel marker expression analysis, each matched set of breast tissues was subjected to molecular signature analysis. This entailed real-time PCR assays using primers specific to a suite of prepublished breast cancer molecular markers against each tissue cDNA. These markers have been used due to their proposed ability to predict tumour sub-types with diagnostic and predictive significance. The relationship between each molecular marker was determined, tabulated for each sample and used as a reference, against which the novel markers could be compared.

### Results and Discussion - Markers Identified by DDRT-PCR.

### RDX-BC-8

Using differential display, a gene fragment, RDX-BC-8, derived from cDNA populations of matched tissue from a breast cancer donor, was observed to have significant up-regulation in the tumour cDNA population in comparison to the corresponding normal tissue cDNA. The 120-nucleotide product (SEQ ID NO.1) was confirmed as differentially expressed by a real-time PCR assay using fragment-specific primers against the source donor normal and tumour tissue set. EMBL and SWISSPROT databases (European Bioinformatics Institute) were then searched and from these searches, RDX-BC-8 exhibited 100% homology over 120 bp to the 3' end of an Fc receptor-like protein 3 (FcRL3; SEQ ID NO.21), with a total size of 4,728 nucleotides on chromosome 1q23.

According to Aceview gene modelling, FcRL3 has 12 different transcripts, produced by alternative splicing, all with introns and all potentially encoding different protein isoforms. Of the 12 transcripts, 8 overlap the RDX-BC-8 sequence with 100% homology. There are 3 probable alternative promoters and 2 non-overlapping alternative last exons. The transcripts appear to differ by truncation of the 5' end, truncation of the 3' end, presence or absence of 3 cassette exons, common exons with different boundaries, because an internal intron is not always spliced out. The homologue detailed in SEQ ID NO.11 is variant C of this gene. SEQ ID NO.11 is the amino acid sequence of FcRL3-c, one of the transcripts with 100% homology to RDX-BC-8. Alignment of FcRL3 isoforms a, b,c,d,e,f,g and h shows that there is a high degree of conservation in this gene family, with 3' and 5' deletions causing the majority of variance. FcRL3 isoforms a,b,d,e,f,g and h are provided as SEQ ID Nos. 27 to 33, respectively Aceview further predicts that this gene family represents membrane or nuclear receptors.

A detailed real-time expression profile of RDX-BC-8 was undertaken using cDNA populations derived from matched breast and tumour tissue samples donated by a number of patients. Of the samples screened, many exhibited notable increases in expression, indicating that this fragment is a useful molecular marker for the presence of breast tumour. The expression profile is represented in Table 1, below.

**Table 1. Expression profile of RDX-BC-8, showing 2- and 4-fold differences between the normal and tumour breast tissue cDNA samples from a number of donors.**

| | 2 fold difference | | 4 fold difference | |
|---|---|---|---|---|
| RDX-BC-8 Increased in tumour | 8 | 80% | 7 | 70% |
| RDX-BC-8 Increased in normal | 1 | 10% | 1 | 10% |
| RDX-BC-8 No discernable difference | 1 | 10% | 2 | 20% |
| RDX-BC-8 No expression evident | 0 | 0% | 0 | 0% |
| Totals | 10 | 100% | 10 | 100% |

To determine organ specificity, RDX-BC-08 was assayed against cDNA populations derived from a panel of 22 human tissue types by real-time PCR analysis. In addition, assays were performed on a range of ethically approved human tumour samples, obtained through Medical Solutions plc, to ascertain whether the marker was breast tumour specific or a less specific marker for the presence of cancer. cDNA representative of tumours from ovary, testis, colon, stomach, liver, lung, bladder and pancreas were also tested. None of the samples tested displayed any significant expression of this novel marker, in comparison to that observed with the breast cancer samples. The FcRL3 protein has not been implicated in cancer or specifically breast cancer, so increased expression in this tissue provides a novel diagnostic, prognostic or predictive use for this marker.

### RDX-BC-9

RDX-BC-9, discovered through DDRT-PCR using cDNA populations of matched tissue from a breast cancer donor, was also observed to have significant up-regulation in the tumour cDNA population in comparison to the corresponding normal tissue cDNA. The 261-nucleotide product (SEQ ID NO.2) was confirmed as differentially expressed by a real-time PCR assay using fragment-specific primers against the source donor normal and tumour tissue set. EMBL and SWISSPROT (European Bioinformatics Institute) were then challenged, revealing no significant homology to any known genes or proteins in their respective databases. Using the BLAT search engine, however, a 99% homology between RDX-BC-9 and the predicted gene H2C10808.1 (EC predictions) was found, sited on chromosome 2q11. The predicted gene comprises 439 nucleotides, although does not extend as far 3' as RDX-BC-9 (SEQ ID NO.22). The predicted gene contains a Kozak sequence and TATA box, so is presumed to be translationally active and contains a presumed coding region (starting at nucleotide 59) of 93 amino acids (SEQ ID NO.12). Cluster analysis confirmed the high homology between these two transcripts.

A detailed real-time expression profile of RDX-BC-9 was undertaken using cDNA populations derived from matched breast and tumour tissue samples donated by a number of patients. These results represent the mean difference of 6 replicates between the normal and tumour samples. Of those screened, many exhibited notable increases in expression in the tumour samples indicating that this fragment is a useful molecular marker for the presence of breast tumour. The expression profile is represented in Table 2, below. At a 2-fold difference, 82% of the sample sets showed increased expression in tumour tissue, with this reducing to 55% when 4-fold differences were calculated, determined by real-time PCR analysis.

**Table 2. Expression profile of RDX-BC-9, showing 2- and 4-fold differences between the normal and tumour breast cDNA samples from a number of donors.**

| | 2 fold difference | | 4 fold difference | |
|---|---|---|---|---|
| RDX-BC-9 Increased in tumour | 9 | 82% | 6 | 55% |
| RDX-BC-9 Increased in normal | 1 | 9% | 0 | 0% |
| RDX-BC-9 No discernable difference | 1 | 9% | 5 | 45% |
| RDX-BC-9 No expression evident | 0 | 0% | 0 | 0% |
| Totals | 11 | 100% | 11 | 100% |

To determine organ specificity, RDX-BC-9 was assayed against cDNA populations derived from a panel of 22 human tissue types by real-time PCR analysis. Assays were also performed on a range of ethically approved human tumour samples, obtained through Medical Solutions plc, to ascertain whether the marker was breast tumour specific or a less specific marker for the presence of cancer. cDNA representative of tumours from ovary, testis, colon, stomach, liver, lung, bladder and pancreas were also tested. This marker was present in most of the tissue samples tested, so is not breast specific. Its increased expression in a significant number of tumour samples in comparison to their normal tissue counterparts, however, makes this gene a suitable indicator for the presence of breast cancer.

### RDX-BC-29

Another DDRT-PCR product was discovered comprising 115 nucleotides (SEQ ID NO.3). Direct sequencing and re-profiling against the source tissue set confirmed initial differential display expression analysis of this candidate, which showed increased expression in the tumour sample. This fragment was then used to search EMBL and SWISSPROT (European Bioinformatics Institute) databases, showing no homology to known genes. It is, however, 100% homologous over 115bp to a hypothetical gene, KIAA0226, also represented by D86979, located on chromosome 3q29. This gene comprises 6644bp (SEQ ID NO.23) and has a coding sequence starting at nucleotide 137, coding for a presumed protein of 960 amino acids (SEQ ID NO.13). The overlapping sequence of RDX-BC-29 and KIAA0226 is located in the 3' non-coding region, from nucleotides 5862 to 5976. According to Aceview, this predicted protein is most likely to be a nuclear protein and it contains a coiled coil domain (amino acids 496-535). It does not belong to any recognised protein family.

A detailed real-time expression profile of RDX-BC-29 was undertaken using cDNA populations derived from matched breast and tumour tissue samples donated by a number of patients. These results represent the mean difference of 6 replicates between the normal and tumour samples. Of those screened, many exhibited notable increases in expression in the tumour samples, indicating that this fragment is a useful molecular marker for the presence of breast tumour. The expression profile is represented in Table 3, below.

**Table 3. Expression profile of RDX-BC-29, showing 2- and 4-fold differences between the normal and tumour breast cDNA samples from a number of donors.**

| | 2 fold difference | | 4 fold difference | |
|---|---|---|---|---|
| RDX-BC-29 Increased in tumour | 9 | 82% | 7 | 64% |
| RDX-BC-29 Increased in normal | 1 | 9% | 0 | 0% |
| RDX-BC-29 No discernable difference | 1 | 9% | 4 | 36% |
| RDX-BC-29 No expression evident | 0 | 0% | 0 | 0% |
| Totals | 11 | 100% | 11 | 100% |

To determine the expression levels of this putative marker in other non-breast tissue types, a panel of 22 healthy tissue and 8 tumour samples were also assayed. RDX-BC-29 was present in most samples tested, so is not breast cancer specific (data not shown). The significant number of donors, who exhibit a 4-fold increase in expression in the tumour sample, however, indicates that this marker has utility as an indicator for the presence of breast cancer.

### RDX-BC-1 02

Initial DDRT-PCR profiling of this 275 nucleotide fragment (SEQ ID NO.4), which showed increased tumour expression, was confirmed through sequencing and confirmational real-time PCR assays against the source tissue set. This fragment was then used to challenge EMBL and SWISSPROT (European Bioinformatics Institute), showing no homology to known genes. It is, however, 100% homologous over 268bp to a hypothetical gene, AK098833, also represented by FLJ25967, located on chromosome 22q12. This gene comprises 9876bp (SEQ ID NO.24) and has a coding sequence starting at nucleotide 1311, coding for a presumed protein of 102 amino acids (SEQ ID NO. 14). The overlapping sequence of RDX-BC-102 and AK098833 is located at the extreme end of the 3' non-coding region, from nucleotide 9609 onwards. The presumed protein has a cleavable signal peptide (residues 1-61) and Aceview predicts this protein will be secreted. It does not belong to any recognised protein family.

A detailed real-time expression profile of RDX-BC-102 was undertaken using cDNA populations derived from matched breast and tumour tissue samples donated by a number of patients. These results represent the mean difference of 6 replicates between the normal and tumour samples. Of those screened, many exhibited notable increases in expression in the tumour samples, indicating that this fragment is a useful molecular marker for the presence of breast tumour. The expression profile is represented in Table 4, below.

**Table 4. Expression profile of RDX-BC-102, showing 2- and 4-fold differences between the normal and tumour breast cDNA samples from a number of donors.**

| | 2 fold difference | | 4 fold difference | |
|---|---|---|---|---|
| RDX-BC-102 Increased in tumour | 10 | 83% | 8 | 67% |
| RDX-BC-102 Increased in normal | 0 | 0% | 0 | 0% |
| RDX-BC-102 No discernable difference | 2 | 17% | 4 | 33% |
| RDX-BC-102 No expression evident | 0 | 0% | 0 | 0% |
| Totals | 12 | 100% | 12 | 100% |

To determine the tissue specificity of this marker, a panel of 22 healthy human tissue cDNA populations and 8 non-breast tumour samples were assayed by conventional PCR. Expression was detected in 15 of the 30 samples tested, including a number of the tumour samples, so this marker is not particular to breast tissue or tumours in general.

### RDX-BC-105

Direct sequencing and re-profiling of this 321 nucleotide fragment (SEQ ID NO.5) against the source tissue set confirmed initial differential display expression analysis, which showed increased expression in the tumour sample. This fragment was then used to challenge EMBL and SWISSPROT databases (European Bioinformatics Institute), showing 100% homology over 324bp to a Transient Receptor Potential Cation Channel Subfamily C Member 4 Associated Protein (TRPC4AP), which is mapped on chromosome 20q11. This gene comprises 1809bp (SEQ ID NO.25) and has a coding sequence starting at nucleotide 527, coding for a presumed protein of 332 amino acids (SEQ ID NO.15). The overlapping sequence of RDX-BC-105 and TRPC4AP is located in the 5' non-coding region, from nucleotides 23 to 342. According to Aceview, the protein will be located either in the nucleus or the cytoplasm.

A detailed real-time expression profile of RDX-BC-105 was undertaken using cDNA populations derived from matched breast and tumour tissue samples donated by a number of patients. These results represent the mean difference of 6 replicates between the normal and tumour samples. Of those screened, many exhibited notable increases in expression in the tumour samples, indicating that this fragment is a useful molecular marker for the presence of breast tumour. The expression profile is represented in Table 5, below.

**Table 5. Expression profile of RDX-BC-105, showing 2- and 4-fold differences between the normal and tumour breast cDNA samples from a number of donors.**

| | 2 fold difference | | 4 fold difference | |
|---|---|---|---|---|
| RDX-BC-105 Increased in tumour | 5 | 56% | 3 | 33% |
| RDX-BC-105 Increased in normal | 0 | 0% | 0 | 0% |
| RDX-BC-105 No discernable difference | 4 | 44% | 6 | 67% |

| | | | | |
|---|---|---|---|---|
| RDX-BC-105 No expression evident | 0 | 0% | 0 | 0% |
| Totals | 9 | 100% | 9 | 100% |

This was detected in about half of the human panel of samples tested, so is not tissue specific, or limited to tumour samples. The notable increases in expression in breast tumour in the matched tissue sets, however, indicate that this marker is a useful indicator for the presence of a breast tumour. In addition, since not all tumours showed this expression profile, this marker may be a useful tool for the sub-classification of such tumours into groups of prognostic or predictive significance, either singly or as part of a signature panel of markers.

### RDX-BC-192

Initial DDRT-PCR profiling of this 324 nucleotide fragment (SEQ ID NO.6), which showed increased tumour expression, was confirmed through sequencing and confirmational real-time PCR assays against the source tissue set. This fragment was then used to challenge EMBL and SWISSPROT databases (European Bioinformatics Institute), where no homologies to known genes or proteins were found. Furthermore, on submission to the BLAT database, no predicted homologues were found, nor were recognised protein motifs. Although small, translational state screening identified this fragment in the polysomal fraction of a cell line culture, indicating that this is likely to code for a protein. A potential peptide of 37 amino acids was found, notable by its CTG (starting at nucleotide 121) start codon (SEQ ID NO.16). The sequence was mapped to 16p12, showing 100% homology to this chromosomal region, with no overlapping repeats.

A detailed real-time expression profile of RDX-BC-192 was undertaken using cDNA populations derived from matched breast and tumour tissue samples donated by a number of patients. These results represent the mean difference of 6 replicates between the normal and tumour samples and are represented in Table 6.

A significant number (60%) of the sample population (n = 10) indicated increased expression in the tumour sample at a 2-fold difference, indicating that this fragment is useful as a molecular marker for breast cancer. This reduced to 20% at the 4-fold level, but these subtle differences may be important indicators of the presence of a tumour in the breast, or may define different tumour types or stages and thus be of prognostic significance.

When screened against the healthy human tissue and non-breast tumour panel, this marker was expressed in most samples assayed, so is not breast tissue specific, or representative only of tumours (data not shown).

**Table 6. Expression profile of RDX-BC-192, showing 2- and 4-fold differences between the normal and tumour breast cDNA samples from a number of donors.**

| | 2 fold difference | | 4 fold difference | |
|---|---|---|---|---|
| RDX-BC-192 Increased in tumour | 6 | 60% | 2 | 20% |
| RDX-BC-192 Increased in normal | 2 | 20% | 1 | 10% |
| RDX-BC-192 No discernable difference | 2 | 20% | 7 | 70% |
| RDX-BC-192 No expression evident | 0 | 0% | 0 | 0% |
| Totals | 10 | 100% | 10 | 100% |

### RDX-BC-220

Initial differential display profiling indicated that this candidate was expressed less in tumour tissue in comparison to its normal counterpart. Sequencing and re-screening of this 176-nucleotide fragment (SEQ ID NO.7) against its source donor tissue confirmed this expression profile. This fragment was then used to challenge EMBL and SWISSPROT databases (European Bioinformatics Institute), where no homologies to known genes or proteins were found. On submission to the BLAT database, however, RDX-BC-220 was homologous over its entire length to the 3' end of a predicted gene, H6C6674 (EC Gene Predictions), also represented by LOC389393 (Ace-View Gene predictions), mapping on the plus strand of chromosome 6p21. The complete sequence of H6C6674 contains 876 nucleotides (SEQ ID NO.26), with a coding sequence starting at nucleotide 112, coding for a presumed protein of 84 amino acids (SEQ ID NO.17). This protein contains no conserved motifs and does not exhibit homology to any known protein in the above databases.

A detailed real-time expression profile of RDX-BC-220 was undertaken using cDNA populations derived from matched breast and tumour tissue samples donated by a number of patients. Results, represented in Table 7, indicate that this marker is significantly reduced in the tumour sample of over 50% of donor tissue sets at a 4-fold difference between normal and tumour cDNA's and is therefore useful as a molecular marker for breast cancer.

When this candidate was assayed against the human tissue and tumour panel, it was expressed at detectable levels in a number of tissue types, namely brain, skeletal muscle, trachea, uterus, lung and stomach tumour, so although not present in all samples, it cannot be regarded as highly specific.

**Table 7. Expression profile of RDX-BC-220, showing 2- and 4-fold differences between the normal and tumour breast cDNA samples from a number of donors.**

| | 2 fold difference | | 4 fold difference | |
|---|---|---|---|---|
| RDX-BC-220 Increased in tumour | 6 | 16% | 4 | 11% |
| RDX-BC-220 Increased in normal | 21 | 57% | 19 | 51% |
| RDX-BC-220 No discernable difference | 6 | 16% | 10 | 27% |
| RDX-BC-220 No expression evident | 4 | 11% | 4 | 11% |
| Totals | 37 | 100% | 37 | 100% |

### Results and Discussion - Database Mined Novel Markers.

Mining of the National Cancer Institute's Cancer Genome Anatomy Project breast cancer SAGE libraries identified a number of theoretical breast cancer-specific novel fragments. All selected fragments from these databases were then subjected to virtual Northern blot analysis and those only present in breast cancer populations were selected. Detailed BLAST and BLAT analysis determined that these fragments were indeed novel in that they were not homologous to any known genes in the EMBL gene banks. All of these fragments, however, showed a high degree of homology (>98%) to predicted genes, as determined through BLAT analysis. Of over 75 fragments initially selected from the breast cancer libraries, only four (described below) passed the selection criteria used to identify useful molecular markers for cancer.

### RDX-BC-GAP-6

The sequence of this database-mined fragment was subjected to BLAST and BLAT analysis, which determined that it did not represent a known gene, but did overlap the predicted gene H20C268, described by EC Gene prediction, and Smertu, described by Acembly Gene Prediction, mapping to chromosome 20p. EC Gene software predicts H20C268 to be made up of 966 nucleotides (SEQ ID NO.8), 270 of which represent a presumed coding region (starting at nucleotide 579), making up a 90 amino acid peptide (SEQ ID NO.18).

A detailed real-time expression profile of RDX-BC-GAP-6 was undertaken using cDNA populations derived from matched breast and tumour tissue samples donated by a number of patients. These results indicate a reduction in expression in the tumour tissue in comparison to the normal tissue counterparts in many donors. When screened against the human tissue panel and various tumour templates, expression was evident in most samples (data not shown), so this marker is present in tissues other than breast tumour. Furthermore, within breast tissue, there is a bias towards normal tissue in comparison to the associated tumour from the same donor. This differential expression of RDX-BC-GAP-6 within normal and tumour tissue indicates that this fragment is a useful indicator for the presence of, and potentially sub-group, a tumour of the breast.

### RDX-BC-GAP-7.

The sequence of this database-mined fragment was subjected to BLAST and BLAT analysis, which determined that it did not represent a known gene, but did overlap the predicted gene H11C12538, described by EC Gene prediction and Koyna described by Acembly Gene Prediction, mapping to chromosome 11q. EC Gene software predicts H11C12538 to be made up of 543 nucleotides (SEQ ID NO.9). Within this sequence, a 141 nucleotide stretch starting at nucleotide 161 represents a presumed coding region of 47 amino acids (SEQ ID NO.19).

To determine the expression profile of this candidate marker, primers were designed and assayed against a panel of matched breast cancer normal and tumour cDNA templates, through real-time PCR. Results from this series of profiles indicate that RDX-BC-GAP-7 shows reduced expression in the tumour in comparison to normal breast tissue in a number of donors, so is useful as a marker for the presence of a tumour.

When primers for this gene were tested against the human normal tissue panel and a number of non-breast tumour samples, expression was only detected in testis and testis tumour samples, so RDX-BC-GAP-7 appears to be specific to only breast and testis tissue, from the range of samples tested. Therefore, RDX-BC-GAP-7 can be a useful biomarker at the protein level for detection in serum, in addition to its utility in tissue samples through PCR detection. In addition, translational state screening determined that this amplicon was present in the polysomal fraction of a study cell line, indicative of translational activity, so it is likely that that a protein is present for this marker.

### RDX-BC-GAP-23.

This sequence was also from the breast cancer CGAP library and found to be novel when used to challenge the EMBL databases, through BLAST analysis, in that it did not match any known genes or proteins. BLAT analysis, however, determined that this fragment was 99% homologous to 2 identical predicted genes, namely Kleysu (Ace-view predictions) and H18C6270 (EC Gene predictions), mapping on chromosome 18q of the human genome. The nucleotide composition of Kleysu, which is representative of all three homologues, is described in as SEQ ID NO.10, with the coding region for the presumed protein starting at nucleotide 141. This region codes for a protein comprised of 103 amino acids (SEQ ID NO.20), which was also confirmed as novel when used to challenge EMBL databases. Aceview predicts that this protein does not belong to any recognised protein family, does not contain any recognised protein domains and the mRNA is expressed at a low level, which agrees with our expression analysis.

Determination of the expression profile for this candidate marker was performed by real-time PCR using co-excised cDNA templates from normal and tumour tissue of the same donor. Through this analysis, RDX-BC-GAP-23 was found to have increased expression in a number of the tumour samples assayed, in comparison to their normal tissue counterparts (data not shown). This indicates that this fragment is useful as a molecular marker for a tumour. Using normal human tissues and a selection of non-breast tumour samples, this marker was expressed only at low levels in the majority of tissues tested, apart from lung, liver and their associated (non-matched) tumours.

### References

DeRisi, J. L., lyer, V. R. and Brown, P. O. 1997. Exploring the metabolic and genetic control of gene expression on a genomic scale. Science. 278: 680-686.
Grover, P. L. and Martin, F. L. 2002. The initiation of breast and prostate cancer. Carcinogenesis. 23 (7): 1095-1102.
Hames, B. D. and Higgins, S. J., (Editors). 1985. Nucleic Acid Hybridisation. A Practical Approach. IRL Press.
Keen, J. C. and Davidson, N. E. 2003. The biology of breast carcinoma. Cancer. 97 (3-Supplement): 825-833.
Liang, P. and Pardee, A. B. 1992. Differential display of eukaryotic messenger RNA by means of the polymerase reaction. Science. 257: 967-971.
Ma, Xiao-Jun., Ranelle Salunga, J. Todd Tuggle, Justin Gaudet, Edward Enright, Philip McQuary, Terry Payette,Maria Pistone, Kimberly Stecker, Brian M. Zhang, Yi-Xiong Zhou, Heike Varnholt, Barbara Smith, Michelle Gadd, Erica Chatfield, Jessica Kessler, Thomas M. Baer, Mark G. Erlander, and Dennis C. Sgroi. 2003. Gene expression profiles of human breast cancer progression. Proc Natl Acad Sci USA. 100 (10): 5974-5979.
Pritzker, K. P. 2002 Cancer biomarkers: easier said than done. Clin. Chem. 2002 Aug; 48(8):1147-50.
Salodof MacNeil. 2001. From genes to proteins: The FLEXgene consortium. HMS Beagle. 112: on-line journal.
Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO, Botstein D, Eystein Lonning P, Borresen-Dale AL. 2001. Gene expression patterns of breast carcinomas distinguish tumor subclasses with clinical implications. Proc Natl Acad Sci USA. Sep 11;98(19):10869-74.
Srinivas PR, Verma M, Zhao Y, Srivastava S. 2002. Proteomics for cancer biomarker discovery. Clin Chem. Aug;48(8):1160-9.
Strausberg R.L., Feingold E.A., Grouse L.H., Derge J.G., Klausner R.D. 2002. Generation and initial analysis of more than 15,000 full-length human and mouse cDNA sequences. Proc. Natl. Acad. Sci. U.S.A. 99(26):16899-16903.
Sturtevant, J. Applications of differential-display reverse transcription-PCR to molecular pathogenesis and medical mycology. Clin Microbiol Rev. 2000 Jul; 13(3):408-27.
Yousef GM, Scorilas A, Kyriakopoulou LG, Rendl L, Diamandis M, Ponzone R, Biglia N, Giai M, Roagna R, Sismondi P, Diamandis EP. 2002. Human kallikrein gene 5 (KLK5) expression by quantitative PCR: an independent indicator of poor prognosis in breast cancer. Clin Chem. 2002 Aug;48(8):1241-50.
Zong, Q., Schummer, M., Hood, L. and Morris, D. R. 1999. Messenger RNA translation state: the second dimension of high-throughput expression screening. Proc. Natl. Acad. Sci. 96: 10632-10636.

## Claims

1. Use of an isolated polynucleotide identified herein as SEQ ID No. 4, or its complement, or a fragment thereof of at least 15 consecutive nucleotides that hybridises to SEQ ID No.4 (or a complement thereof) under stringent hybridising conditions in an *in vitro* diagnostic assay to test for the presence of or the risk of breast cancer in a patient, wherein an increased expression of the polynucleotide indicates the presence of or the risk of breast cancer in the patient.

2. Use of a polynucleotide that hybridises with or inhibits the expression of a polynucleotide as defined in claim 1, in the manufacture of a medicament for the treatment of breast cancer.

## Patentansprüche

1. Verwendung eines hierin als SEQ ID NO: 4 identifizierten isolierten Polynukleotids, oder dessen Komplement, oder ein Fragment davon aus mindestens 15 konsekutiven Nukleotiden, das unter stringenten Hybridisierungsbedingungen in einem diagnostischen *In-vitro*-Test zum Testen auf das Vorliegen von Brustkrebs oder des Risikos für Brustkrebs bei einem Patienten/einer Patientin an SEQ NO: 4 (oder ein Komplement davon) hybridisiert, wobei eine erhöhte Expression des Polynukleotids auf das Vorliegen von Brustkrebs oder des Risikos für Brustkrebs bei dem Patienten/der Patientin hindeutet.

2. Verwendung eines Polynukleotids, das wie nach Anspruch 1 definiert mit einem Polynukleotid hybridisiert oder dessen Expression inhibiert, bei der Herstellung eines Arzneimittels zur Behandlung von Brustkrebs.

## Revendications

1. Utilisation d'un polynucléotide isolé identifié dans le présent document comme correspondant à la SEQ ID No. 4, ou à son complément, ou d'un fragment de celui-ci composé d'au moins 15 nucléotides consécutifs qui s'hybride à la SEQ ID No. 4 (ou à un complément de celle-ci) sous conditions d'hybridation rigoureuses dans le cadre d'un dosage diagnostique *in vitro,* en vue de tester la présence de, ou le risque de, cancer du sein chez une patiente, dans laquelle une plus forte expression du poynucléotide indique la présence de, ou le risque de, cancer du sein chez la patiente.

2. Utilisation d'un polynucléoide qui hybride avec ou inhibe l'expression d'un polynucléotide tel que défini à la revendication 1, dans la fabrication d'un médicament pour le traitement du cancer du sein.
